# EUROPEAN PATENT SPECIFICATION

(11) **EP 4 366 689 B1**
(45) Date of publication and mention of the grant of the patent: **02.09.2026**
(21) Application number: 22747755.1
(22) Date of filing: 06.07.2022
(51) Int. Cl.: A61K 35/747, A61K 8/73, A61K 8/99, A61P 17/00, A61Q 17/04, A61Q 19/00

(54) **LACTICASEIBACILLUS PARACASEI LIVESKIN88 (DSM 33788) AND ITS USES IN THE TREATMENT OF SKIN INFECTIONS AND IN COSMESIS**
LACTICASEIBACILLUS PARACASEI LIVESKIN88 (DSM 33788) UND DESSEN VERWENDUNG BEI DER BEHANDLUNG VON HAUTINFEKTIONEN UND IN DER KOSMETIK
LACTICASEIBACILLUS PARACASEI LIVESKIN88 (DSM 33788) ET SES UTILISATIONS DANS LE TRAITEMENT DES INFECTIONS CUTANÉES ET EN COSMÉTIQUE

(30) Priority: 06.07.2021 IT 202100017855
(43) Date of publication of application: 15.05.2024
(73) Proprietor: LAC2BIOME S.r.l., 20154 Milano (IT)
(72) Inventor: BIFFI, Andrea, 20154 Milano (MI) (IT)
(74) Representative: Benedetto, Marco
(86) International application number: PCT/IB2022/056244
(87) International publication number: WO 2023/281415

(56) References cited:
- WO-A1-2019/111189
- WO-A1-2020/245797
- WO-A1-2021/053639
- WO-A1-2021/053642
- WO-A1-2022/208458
- SILVIA BALZARETTI ET AL: "The vaginal isolate Lactobacillus paracasei LPC-S01 (DSM 26760) is suitable for oral administration", FRONTIERS IN MICROBIOLOGY, vol. 6, 15 September 2015 (2015-09-15), XP055474499, DOI: 10.3389/fmicb.2015.00952
- ALICE CASARI ET AL: "Evaluation of A Novel Topical Formulation for The Treatment of Problematic Skin: Results of An Italian Clinical Study", JOURNAL OF DERMATOLOGY RESEARCH AND THERAPY, vol. 8, no. 2, 1 January 2022 (2022-01-01), pages 1152469 - 5750, XP055977640, Retrieved from the Internet <URL:https://clinmedjournals.org/articles/ijdrt/journal-of-dermatology-research-and-therapy-ijdrt-8-115.pdf?jid=ijdrt> DOI: 10.23937/2469-5750/1510115

## Description

The present invention relates to selected bacterial strains, belonging to the *Lactobacillus paracasei* species (recently reclassified by J. Zheng et al., 2020), and mixtures thereof, to their compositions and their use in the prevention and/or treatment of skin diseases and impairments.

### BACKGROUND

Numerous types of topical skin care products are available like cleansers, toners, moisturizers, anti-aging serums, etc. These compositions very often contain chemicals that are relatively aggressive to the skin and can in the long term give rise to pronounced damages to the skin. These products usually contain chemical ingredients that may independently or in combination with other ingredients harm the skin instead of benefiting it and are, therefore, not suitable for all types of skin. Moreover, these formulations may cause irritation to certain skin types.

Therefore, there is a strong demand for the identification of new treatments for the skin care capable of reducing skin imperfections, irritations/redness, dryness, and/or inflammations of skin as alternatives to conventional chemical cosmetic compositions and treatments.

The Applicant, following intensive and prolonged research and development, has surprisingly found out that specific bacterial strains of the genus *Lactobacillus* belonging to the species *Lactobacillus paracasei* and identified as *Lactobacillus paracasei* LPC-S01^{®} and deposited with the *Deutsche Sammlung von Mikroorganismen und Zellkulturen GmbH* (DSMZ) under deposit number DSM 26760 by Sofar S.p.A. on January 11, 2013, and converted to a deposit under the Budapest treaty on May 15, 2017, can solve the above mentioned technical problem.

The Applicant, following an intensive and prolonged research and development, has surprisingly found out that specific bacterial strains of the genus *Lactobacillus* belonging to the species *Lactobacillus casei spp.* casei identified as *L. casei* DG^{®} and deposited with the *Collection Nationale de Cultures de Microorganismes* INSTITUT PASTEUR (CNCM) under deposit number CNCM I-1572 by Sofar S.p.A. on May 5, 1995, can solve the above technical problem.

The Applicant, following an intensive and prolonged research and development, has surprisingly found out that another specific bacterial strain identified as *Lacticaseibacillus paracasei* m.biome LIVESKIN88 (ex *Lactobacillus paracasei* or *L. paracasei*) and deposited with the Deutsche Sammlung von Mikroorganismen und Zellkulturen GmbH (DSMZ) as *Lactobacillus paracasei* under deposit number DSM 33788 (filed on January 20, 2021 by LAC2BIOME S.r.l., Italy; subsequently renamed as *Lacticaseibacillus paracasei* DSM) 33788, can also solve several skin impairments.

Said strains of bacteria have been deposited in accordance with the provisions of the Budapest Treaty; the depositor of said strains of bacteria described and claimed in this patent application and the applicant express their consent to make the strains available for the duration of the patent.

### BRIEF DESCRIPTION OF THE FIGURES

Figure 1: adhesion of *P. acnes* in terms of percentage of living and vital cells adherent following the contact pre-stimulation of the keratinocytes with the different probiotics tested (in the figure, (*) statistically significant data (p<0.05)). LP125=LPC-S01; LC48=DG
Figure 2: adhesion of *P. acnes* in terms of percentage of living and vital cells adherent following co-incubation of keratinocytes with *P. acnes* and with the different probiotics tested (in the figure, (*) statistically significant data (p<0.05)).
Figure 3: adhesion of *P. acnes* in terms of percentage of living and vital cells adherent following the incubation of keratinocytes with the different probiotics tested, after challenge of eukaryotic cells with the pathogen. (in the figure, (*) statistically significant data (p<0.05)).
Figure 4: immunomodulatory effect on IL1β, IL10 and IL8 by probiotic strains.
Figure 5: western blotting on COX-2 and NF-kB.

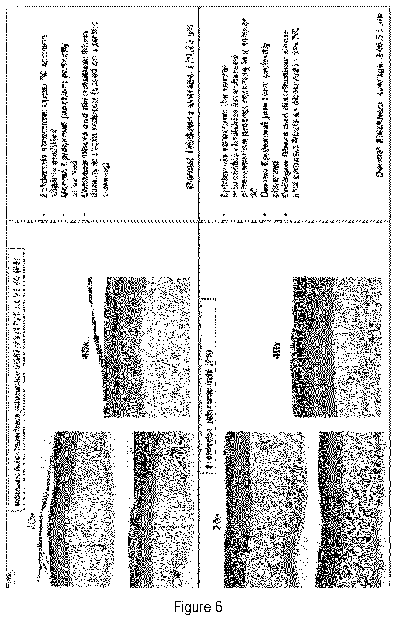

Figure 6: dermal thickness average measurements.

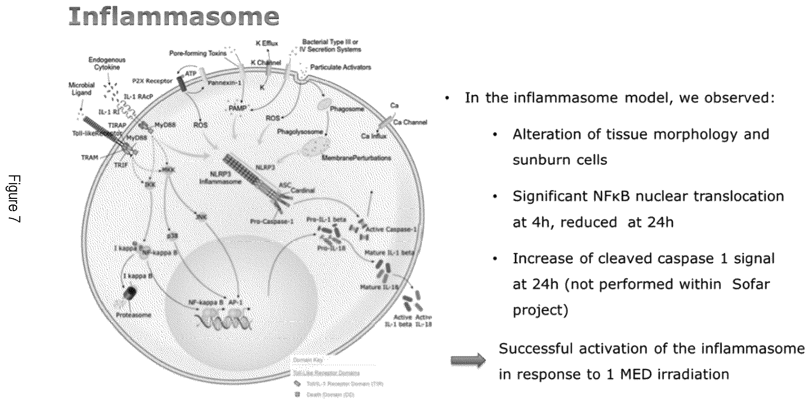

Figure 7: drawing of the Inflammasome model.
Figure 8: blocks diagrams reporting the VS 42-18 NF-kB Translocation at 4h. CN: Negative control, CP: Positive control, P1: LPC S01, P2: Hyaluronic acid mask + LPC S01, P3: Hyaluronic acid mask.
Figures 9 and 10: Inflame-Aging Model on T-skin at 4 hours and 24 hours.
Figure 11A and Figure 11B: gene expression results after 24h (left) and after 48h (right). RQ calculated with NC 24h=1, RQ calculated with NC 48h=1. RQ < 0.5 down regulation, RQ> 2 up regulation.
Figure 12: skin structure.
Figures 13-15: protocols of the study 4b.2. Figure 14 represents Pre-treatment protocol; Figure 15 represents the post-treatment protocol.
Figure 16A and Figure 16B: reduction of the viability of *C. acnes* (formerly *P. acnes*) DSM 1897 expressed in Log10 CFUs/insert (left part of Figure 16). On the right, percentage of reduction in viability of *C. acnes* DSM 1897 in the various conditions tested in the pre-treatment model with probiotic and subsequent infection with pathogen.
Figure 17: panel test.
Figure 18A and Figure 18B: reduction of the viability of *C. acnes* (formerly *P. acnes*) DSM 1897 expressed in Log10 CFUs/insert (left part of Figure 16). On the right, percentage of reduction in viability of *C. acnes* DSM 1897 in the various conditions tested in the competition test.
Figure 19A and Figure 19B: reduction of the viability of *C. acnes* (formerly *P. acnes*) DSM 1897 expressed in Log10 CFUs/insert (left part of Figure 16). On the right, percentage of reduction in viability of *C. acnes* DSM 1897 in the various conditions tested in the displacement test.
Figure 20: shows skin surface hydration value of the treated area obtained on the 29 volunteers.
Figure 21: represents the clinical evaluation of the extension of the facial area involved and shows delta values obtained on the 29 volunteers.
Figure 22: shows delta values obtained on the 29 volunteers and shows a statistically significant decrease regarding the erythema.
Figure 23: shows delta values obtained on the 29 volunteers and shows a statistically significant decrease regarding the number of papules and/or pustules.
Figure 24: shows delta values obtained on the 29 volunteers and shows a statistically significant decrease regarding the skin dryness.
Figure 25: shows the results of the treatment based on 29 cases per sample after 14-days *versus* 28-days treatment.
Figure 26 shows Ha mask + *Lacticaseibacillus paracasei* m.biome *LIVESKIN88* (DSM 33788) promoted epithelial cells proliferation. Quantification of the wound clousure in HaCaT cells after incubation at different time point, using different dilution of complete mask.
Figure 27 shows the quantification epithelization using different bacterial cells concentration resuspended in 1 to 20 excipients mask diluted in water.
Figure 28 shows the adhesion of *Lacticaseibacillus paracasei* m.biome *LIVESKIN88* (DSM 33788) strain to the HaCaT epithelial cell layer as observed with Giemsa staining under a light microscope.
Figure 29 shows the study of the activation of the NF-κB transcriptional regulator in a HaCaT cell layer transfected with an alkaline phosphatase (SEAP) reporter vector.
Figure 30 shows the comparison of scavenging effect of different probiotic preparation.
Figure 31 shows the intracellular ROS modulation in HaCaT cells by antioxidant effect of *Lacticaseibacillus paracasei* m.biome *LIVESKIN88* (DSM 33788).
Figure 32 shows the antipathogenic effect of *Lacticaseibacillus paracasei* m.biome *LIVESKIN88* (DSM 33788) preparation in comparison with different *Lactobacillus* suspensions.

### DESCRIPTION

The invention is defined by the appended claims.

It was interestingly found that two particular strains of bacteria are highly effective on several skin diseases and impairment by exerting a specific action on *P. acnes.*

According to a first aspect thereof, the description relates to a strain of bacteria for use in the preventive or curative treatment of infections and/or inflammations caused by pathogenic bacteria belonging to the species *Propionibacterium acnes;* wherein said strain is selected from the group consisting or, alternatively, comprising of:
- *Lactobacillus paracasei* LPC-S01 (DSM 26760)
- *Lactobacillus casei* DG^{®} (CNCM I-1572), and mixtures thereof;
characterized in that said preventive or curative treatment is exerted *via* a specific action on *P. acnes.*

According to another of its aspects, the description relates to a composition comprising at least one of the above strains and optionally at least a pharmaceutically acceptable excipient, characterized in that said composition is intended to inhibit inflammation *via* a specific action on *P. acnes.*

According to another of its aspects, the description relates to a cosmetic method for the treatment of acne, comprising administering a bacterial strain selected from the group comprising or, alternatively, consisting of:
- *Lactobacillus paracasei* LPC-S01 (DSM 26760)
- *Lactobacillus casei* DG^{®} (CNCM I-1572), and mixtures thereof;
characterized in that said method is intended to inhibit inflammation *via* a specific action on *P. acnes*

According to a preferred embodiment, the description relates to the cosmetic method above, wherein a bacterial strain selected from the group comprising or, alternatively, consisting of:
- *Lactobacillus paracasei* LPC-S01 (DSM 26760)
- *Lactobacillus* casei DG^{®} (CNCM I-1572), and mixtures thereof,
is administered to the person in need once per day.

The use of at least a bacterial strain as defined above, for the preparation of a cosmetic, or dermatological composition for treating or preventing acne, characterized in that said cosmetic or dermatological composition acts *via* a specific action on *P. acnes,* represents another subject-matter of the description.

According to another of its aspects, the description relates to a composition comprising:
- a bacterial strain selected from the group comprising or, alternatively, consisting of: *Lactobacillus paracasei* LPC-S01 (DSM 26760), *Lactobacillus casei* DG^{®} (CNCM I-1572), and mixtures thereof
- hyaluronic acid or its salts thereof;
characterized in that the composition is intended to inhibit inflammation *via* a specific action on *P. acnes*

According to another of its aspects, the description relates to a cosmetic method for enhancing skin hydration, comprising:
- applying the composition as above defined on the skin and maintain the application for at least 6 hours;
- removing the applied composition with water,
characterized in that the application is carried out for at least 14 days, preferably for a period comprised from 14 to 28 days.

According to another of its aspects, the description relates to a kit comprising:
- the composition as above defined; and

- a dispenser having a separate compartment for the bacterial strain.

According to another of its aspects, the description relates to a composition comprising:
- a bacterial strain selected from the group comprising or, alternatively, consisting of: *Lactobacillus paracasei* LPC-S01 (DSM 26760), *Lactobacillus casei* DG^{®} (CNCM I-1572), and mixtures thereof
- hyaluronic acid or salts thereof;
for use in protecting human skin from ultraviolet radiation and enhancing skin hydration.

Object of the invention relates to a composition comprising:
- *Lacticaseibacillus paracasei* m.biome *LIVESKIN88* (DSM 33788)
- hyaluronic acid or salts thereof;
for use in wound healing and in promoting epithelization.

According to another of its aspects, the invention relates to the use of a dermatological or cosmetic composition comprising:
- *Lacticaseibacillus paracasei* m.biome *LIVESKIN88* (DSM 33788); and optionally
- hyaluronic acid or salts thereof;
in preventing skin ageing.

According to another of its aspects, the invention relates to a strain of bacteria for use in the preventive or curative treatment of skin infections and/or skin inflammations caused by *Streptococcus aureus;* wherein said strain is *Lacticaseibacillus paracasei* m.biome *LIVESKIN88* (DSM 33788).

According to another of its aspects, the invention relates to a composition comprising *Lacticaseibacillus paracasei* m.biome *LIVESKIN88* (DSM 33788) and optionally at least a pharmaceutically acceptable excipient, for its use in the preventive or curative treatment of skin infections and/or skin inflammations caused by *Streptococcus aureus.*

The bacterial strains and the composition of the invention are intended for topical use, one or more times per day, preferably once per day.

Preferably, the strains of the invention are in lyophilized form and are administered in pharmaceutical, dermatological or cosmetic compositions for topical use, optionally in presence of conventional excipients. Such compositions can be medicaments or cosmetic products and can be prepared according to the methods known in the art and can be in a dosage unit form or a multiple dosage form. For instance, the compositions of the invention can be prepared under the form of gel, creams, emulsions, ointments, foams, powders, aqueous solutions or suspensions, oil solutions or suspensions or also biphasic solutions or suspensions, to be stirred before use. Masks are also suitable for the treatment of the invention.

The compositions can contain from 10³ to 10¹², for instance from 10⁵ to 10¹⁰CFU (colony-forming units) per gram of composition. Such compositions could be administered in appropriate amounts to the extent of the area to be treated. The treatment is preferably extended during at least 14 days and preferably until the dermatological or cosmetic result desired is achieved
The compositions of the invention, beside conventional excipients, may also contain further active ingredients, beneficial to the treatment of the disease or cosmetic impairment. As an alternative, the administration of the strains or composition of the invention can also be carried out in connection with another convenient treatment, either topical or parenteral.

### EXPERIMENTAL SECTION

### Probiotics vs P. acnes (Cutibacterium acnes, formerly Propionibacterium acnes)

Two strains of *Lactobacillus paracasei,* namely *L. paracasei* LPC-S01 and *L. casei* DG in order to evaluate their effectiveness in:
- inhibiting the growth of *P. acnes;*
- modulating the inflammatory reaction of keratinocytes following challenging with inflammatory stimulation (LPS).

To perform these experiments normal human keratinocytes have been used, kept in culture with the appropriate supplements.

Cultured human keratinocytes have been identified as a suitable substrate for the preliminary determinations necessary to evaluate the efficacy of a probiotic intended for application in the mitigation of skin irritations. The keratinocytes represent in fact the first line of defence of the skin with respect to the external environment, being disseminated in the external cutaneous layer (epidermis) and can induce the secretion of cytokines and chemokines to convey the alert message to the deeper layers of the skin, generating the inflammatory response. During their evolution they suffer a migration from the deepest layers to the most superficial ones, with progressive deposition of keratin, responsible for the protective action.

Human primary keratinocytes can be cultured in-vitro in the laboratory and destined for culture testing with bacterial strains in order to identify the nature of the immune response from the latter induced.

The bacterial strains used are listed below:
- L. *casei* DG^{®} (*L. paracasei* CNCM I-1572)
- *L. paracasei* LPC-S01^{®} (DSM 26760)
- L. *casei* DG^{®} *+ L. paracasei* LPC-S01, 1:1 mixture

The following tests have been conducted:
1. Adhesion and challenging tests of the cell line with the pathogen;
2. Immuno-modulation test;

### 1.2.1 Adhesion and challenging tests

These experiments are to verify the ability of specific strains of *Lactobacillus paracasei,* alone or in a 1:1 mixture, to interfere with the adhesion of *Propionibacterium acnes* on normal human keratinocytes in culture.

In the exclusion test (pre-treatment of eukaryotic cells with probiotics and subsequent incubation with the pathogen), the results have shown that both probiotic strains showed an ability to prevent adhesion of *P. acnes,* in similar % (42% for *L. casei* DG^{®} and 35% for *L. paracasei* LPC-S01).

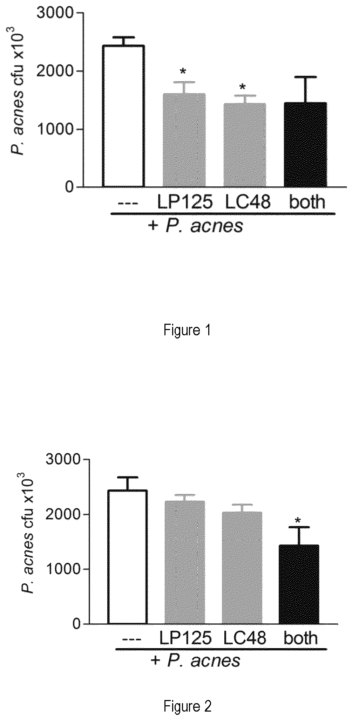

The results are reported in Figure 1 which represents the adhesion of *P. acnes* in terms of percentage of living and vital cells adherent following the contact pre-stimulation of the keratinocytes with the different probiotics tested. After the adhesion of *P. acnes* in the absence of stimulation by probiotics, the inhibition capacity of the tested strains is expressed as % of adhesion reduction by *P. acnes* compared to the positive control.

In the competition test (co-incubation of eukaryotic cells with probiotics and with the pathogen), the results tests have shown that the *L. casei* DG^{®} strain has an adhesion reduction capacity of 17% while the *L. paracasei* LPC-S01 strain has an adhesion reduction capacity of 9%. The mixing of the strains produced a statistically significant reduction of *P. acnes* adhesion, reaching 42%, a percentage clearly higher than that observed for the strains considered individually, clearly showing a synergistic effect. The results are reported in Figure 2.

Figure 2 represents the adhesion of *P. acnes* in terms of percentage of living and vital cells adherent following co-incubation of keratinocytes with *P. acnes* and with the different probiotics tested. As previously described, with the adhesion of *P. acnes* in the absence of stimulation by probiotics, the inhibiting capacity of the tested strains was expressed as % of adhesion reduction by *P. acnes* compared to the positive control.

In the displacement test (pre-treatment of eukaryotic cells with the pathogen and subsequent incubation with the probiotic), the results have shown an interesting statistically significant synergistic effect was observed, linked to the mixture of 2 probiotics which proved to be able to reduce the adhesion of *P. acnes* of 42 %. The individual probiotics have instead revealed adhesion reduction capacity equal to 18% for *L. casei* DG^{®} and 11% for *L. paracasei* LPC-S01.

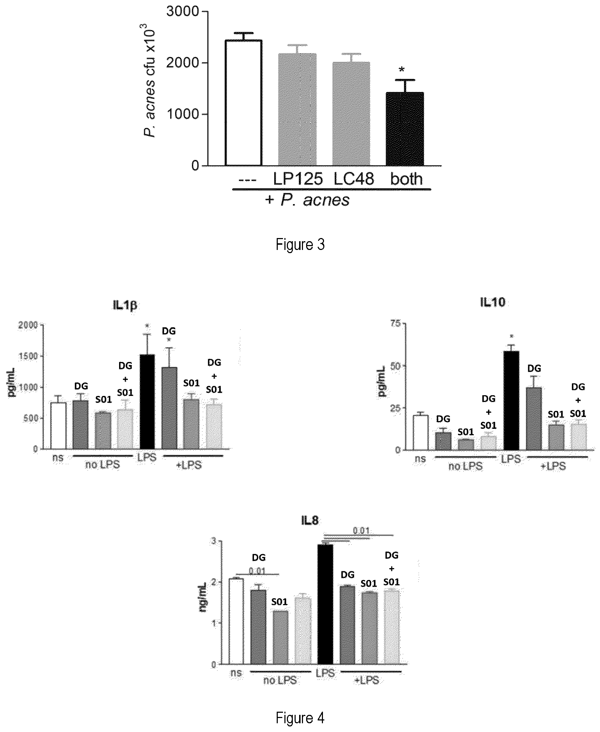

Results are reported in Figure 3 which represents the adhesion of *P. acnes* in terms of percentage of living and vital cells adherent following the incubation of keratinocytes with the different probiotics tested, after challenge of eukaryotic cells with the pathogen. As previously described, with the adhesion of *P. acnes* in the absence of probiotics, the inhibiting capacity of the tested strains was expressed as % of adhesion reduction by *P. acnes* compared to the positive control.

### 1.2.2 Immuno-modulation test

The objective of these experiments was to verify the ability of specific strains of *Lactobacillus paracasei,* alone or in a 1:1 mixture, to exert an immuno-modulating effect on normal human keratinocytes in culture. The tests were focused on the determination of cytokines (IL-8, IL-1beta, and IL-10) and on the evaluation of activation of 2 markers COX-2 and NF-kB.

COX-2 (cyclooxygenase-2) represents an inducible marker, produced by a small number of cell types in response to a specific inflammatory stimulus. It appears to be over-expressed in several neoplasms, including skin ones.

NF-kB (nuclear factor kappa-light-chain-enhancer of activated B cells) is a protein complex with transcription factor function, produced by all cell types in response to various stimuli, including those of inflammatory nature.

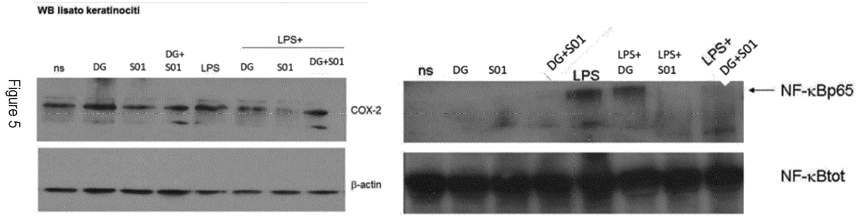

The results have shown as follows:
- The cytokine assay in the supernatant of cells exposed to probiotics allowed to highlight an immunomodulatory effect, reducing the expression of IL1β, IL10 and IL8 by probiotic strains considered individually, with particular reference to *L. paracasei* LPC-S01. Results are reported in Figure 4.
- The immunomodulatory effect following LPC-S01-induced activation is also confirmed by the results of western blotting on COX-2 and NF-kB. A particular potential is associated with the *L. paracasei* LPC-S01 strain although the L. *casei* DG^{®} strain has also been interesting but less efficient in the overall anti-inflammatory action against keratinocytes. Results are shown in Figure 5.

### 1.3 Summary of the results

The synergistic potential of the mixed strains is significant in the displacement of the pathogen, if the mixture is used together with the presence of the pathogen in contact with the keratinocytes or subsequently to the infection, for the containment of its proliferation.

From an immunomodulatory point of view, in many cases the *L. paracasei* LPC-S01 strain was more effective than L. *casei* DG^{®} in modulating the inflammatory reaction of keratinocytes following challenging with inflammatory stimulation (LPC-S01).

### 2. Probiotics + mask - homeostatic model

The aim of the study was to evaluate a series of samples based on hyaluronic acid, or collagen, or jojoba oil, with or without the probiotic strains according to the invention. The studies have been performed on a complete 3D model reconstituted *in vitro,* including the dermis and epidermis (Full Thickness skin model), in order to explore their potential application and efficacy for skin care.

### 2.1 Background

*In vitro* reconstructed human skin models are closer in term of morphology (multi-stratified epithelium), biochemical and physiological properties to *in vivo* human tissues and represent today the most promising alternative to animals, *ex vivo* explants and submerged cell monolayers for *in vitro* safety and efficacy evaluation of topically applied products (Gordon et al. 2015, Zuang V. 2016).

The biological relevance and predictivity of these models derive from the presence of an organized tissue with different living cell layers allowing to assess the products topically at realistic clinical doses and exposure conditions.

The treatment of human skin with topically applied products, such as cosmetics, leads to a genomic response which has a dynamic pathway and represents the first cellular signal at transcriptional level responsible for a cascade of events. 3D living human tissues are relevant test systems to investigate the mechanism of action and to assess product efficacy taking into account both the direct genomic response and the results of cellular communication and crosstalk via soluble mediators and specific biomarkers expression.

It was developed a specific model for cosmetic product testing a homeostasis model on "Full-thickness skin model" (T-skin) reproducing dermal and epidermal compartments: this model has the specificity to allow the study of dermal extracellular matrix modification and viable layer differentiation with a multi-parametric approach.

### 2.2 The study

A series of new products based on hyaluronic acid, collagen, or jojoba oil in a reference formulation ("Mask") as such and in presence of the probiotic strain LPC-S01 were evaluated on a complete 3D model reconstituted *in vitro,* including the dermis and epidermis (Full Thickness skin model), in order to explore their potential application and efficacy for skin care.

The tested products are as follows:

**Table 1**

| NAME | LABELLED AS |
|---|---|
| Probiotic strain LPC-S01 | P1 |
| Collagen mask | P2 |
| Hyaluronic acid mask | P3 |
| Jojoba Oil mask | P4 |
| LPC-S01+Collagen mask | P5 |
| LPC-S01+hvaluronic acid mask | P6 |
| LPC-S01+Jojoba Oil mask | P7 |

The products were applied directly on the surface of the 3D model for 8h in a physiological daily exposure, followed by a gentle washing procedure with saline solution to remove the product excess, and a post-incubation of 16h in order to mimic a realistic exposure of a mask.

The following parameters have been considered on the complete 3D model reconstituted in vitro, including the dermis and epidermis (FT-skin model), in order to define their skin tolerance and their efficacy in:
• enhancing skin auto-defence by induction of anti-microbial peptides;
• stimulating keratinocytes innate immuno-response, epidermal renewal and differentiation;
• inducing a positive renewal of the epidermal and dermal compartments, acting as antiaging.

**Table 2**

| AIM | BIOMARKER/PARAMETER |
|---|---|
| Skin tolerance | Quantification of cytotoxicity by Toxilight assay |
| Enhancing skin auto-defence by induction of anti-microbial peptides | HBD2 and CCL27 gene expression |
| Stimulating keratinocytes innate immune-response, epidermal renewal and differentiation | TLR-2, KRT14, loricrin and involucrin gene expression |
| Inducing positive renewal of the epidermal and dermal compartments, acting as antiaging | *•* HAS-2 (hyaluronate synthetase) and CD44 (hyaluronic acid receptor) |
| | *•* Collagens III and IV (dermis) and XIII (epidermis) |
| | *•* KGF and EGF (keratinocytes growth factors) |
| | by gene expression |
| | Trichrome Masson by histological analysis including the measure of epidermal and dermal thickness |

The main positive results obtained are reported in the following table for each product, by comparing for the 3 main ingredients (jojoba oil, hyaluronic acid, collagen) in the different mask-based products, with or without the bacterial strain *Lactobacillus paracasei* LPC-S01.

**Table 3**

| PRODUCTS | | MAIN POSITIVE RESULTS |
|---|---|---|
| | LPC-S01 (P1) | • Up regulation of HBD2 and TLR-2 → immunomodulatory effect, triggering the skin defences and boosting the innate response |
| | | • Preservation of tissue morphology → good biocompatibility |
| COLLAGEN | P2 | - |
| | +LPC-S01 (P5) | • Boosting of HBD2 → increased skin innate defence |
| HYALURONIC ACID | P3 | • DEJ structure has not been significantly modified |
| | +LPC-S01 (P6) | • Boosting of HBD2 indicating increased skin defences |
| | | • Enhanced differentiation and increases stratum corneum compactness |
| | | • Increase collagen network |
| JOJOBA OIL | P4 | - |
| | +LPC-S01(P7) | • Boosting of HBD2 → increased skin defences |
| | | • Partial preservation of DEJ and collagen network |

The best results have been obtained with the combination of hyaluronic acid and the strain *L. paracasei* LPC-S01.

For the morphological analysis, a scoring of qualitative evaluation with respect to the control has been given:
+ → no significant modifications
+ + → significant improvement of morphological descriptors
- → slightly altered morphology
- - → altered morphology

**Table 4**

| PRODUCTS | | BIOLOGICAL EVIDENCES | MORPHOLOGICAL EVIDENCES | | |
|---|---|---|---|---|---|
| | LPC-S01 (P1) | • Down-regulation of Collagen III and HAS-2 | + | + | - |
| COLLAGEN | P2 | • Genes under investigation not modulated | - | - | + |
| | +LPC-S01 (P5) | • Down-regulation of HAS-2 and KGF • Reduced Collagen III expression | + | - | - |
| HYLURONIC ACID | P3 | • Genes under investigation not modulated | - | ++ | + |
| | +LPC-S01 (P6) | • Down-regulation of Collagen III, HAS-2 and KGF | ++ | ++ | + |
| JOJOBA OIL | P4 | • Up-regulation of TLR-2 and increased AK release during the treatment potential activation of inflammatory response | -- | -- | - |
| | | • Down-regulation of loricrin and histological modifications of basal layers → potential alteration of the differentiation process | | | |
| | +LPC-S01 (P7) | • Down-regulation of Collagen III | -- | + | |

### 2.3 Summary of the results

Globally, the results obtained indicate that the probiotic LPC-S01 exerts a positive effect on the skin by enhancing its innate immunity (based on TLR 2 and HBD-2) when applied alone.

The products based on hyaluronic acid, or collagen, or jojoba oil without the probiotic strain have not exerted a positive effect on the T-skin model; on the contrary, the same products also comprising the bacterial strain LPC-S01 show a positive effect in enhancing the skin enhancing skin auto-defence.

The product hyaluronic acid mask + *Lactobacillus paracasei* LPC-S01 resulted as the most promising combination showing a positive efficacy:
- in enhancing the skin differentiation process and skin renewal;
- in boosting globally the dermal compartment structure by increasing the collagen network.

### 3a. Probiotics + mask - inflammasome model - live cells

### Aim

With this study was evaluated the effect of the strain *Lactobacillus paracasei* LPC-S01, alone or in combination with the hyaluronic acid ("the Mask"), on an inflammasome model in order to explore their potential application and efficacy for inflammation reduction. This study was focused on the product effect on inflammasome activation in response to UV irradiation and thus inflammation.

**Table 5**

| AIM | BIOMARKER/PARAMETER |
|---|---|
| Prevention of inflammasome activation | • Evaluation of NF-kB nuclear translocation |
| | • Quantification of secreted IL-1β |
| Reduction of the UV damages at epidermal and dermal level | *•* Evaluation of dermal and epidermal morphology by hematoxylin and eosi staining |

### 3a.1 Background

*In vitro* reconstructed human skin models are closer in term of morphology (multistratified epithelium), biochemical and physiological properties to *in vivo* human tissues and represent today the most promising alternative to animals, ex vivo explants and submerged cell monolayers for in vitro safety and efficacy evaluation of topically applied products (Gordon et al. 2015, Zuang V. 2016).

The biological relevance and predictivity of these models derive from the presence of an organized tissue with different living cell layers allowing to assess the products topically at realistic clinical doses and exposure conditions. The treatment of human skin with topically applied products, such as cosmetics, leads to a genomic response which has a dynamic pathway and represents the first cellular signal at transcriptional level responsible for a cascade of events.

3D living human tissues are relevant test systems to investigate the mechanism of action and to assess product efficacy taking into account both the direct genomic response and the results of cellular communication and crosstalk via soluble mediators and specific biomarkers expression.

In the present study the "Full-thickness skin model" (T-skin) reproducing dermal and epidermal compartments, specifically developed for cosmetic products testing by VitroScreen was used. This model has allowed the study of dermal extracellular matrix modification and epidermal differentiation with a multi-parametric approach in a stress condition induced by UV radiation.

### 3a.2 The study

A new product based on hyaluronic acid in a reference formulation ("Mask") as such and in presence of a probiotic bacterial strain *Lactobacillus paracasei* LPC-S01 was evaluated on an inflammasome model in order to explore their potential application and efficacy for inflammation reduction. The products tested are as follows:

**Table 6**

| NAME | INTERNATIONAL CODE |
|---|---|
| Probiotic strain LPC-S01 | P1 |
| LPC-S01 + hyaluronic Acid mask | P2 |
| Hyaluronic acid mask | P3 |

The products were applied directly on the surface of the 3D model and incubated overnight, followed by a gentle washing procedure with saline solution to remove the
product excess. The tissues were slightly abraded and then exposed to 1 MED of UV to mimic normal sun exposure. The activation of the inflammasome pathway was assessed 4h and 24h after 1 MED irradiation.

The following parameters have been analysed compared to untreated control:
- Immunostaining of NF-κB
- Hematoxylin and Eosin staining
- Quantification of secreted IL-1β by ELISA

The positive control (inflammasome induced by 1 MED) displayed the following features:

**Table 7**

| Early time point: 4h | Late time point: 24h |
|---|---|
| • Increase of NFKB nuclear translocation | *•* Decrease of NFKB in the tissue, no more nuclear translocation |
| • Presence of multiple sunburn cells | |

In term of morphology, all irradiated tissues presented sunburn cells at 4h followed by significant epidermal and dermal damages at 24h as expected after epidermal abrasion.

The main results of test items obtained are reported in the following table, by comparing for the mask (hyaluronic acid), the probiotic strain LPC-S01 and the combination of both compared to positive irradiated control.

**Table 8**

| PRODUCT | MAIN RESULTS |
|---|---|
| LPC-S01 (P1) | • Decrease of nuclear translocation (4h) |
| | • Decrease of cytoplasmic NFKB (24h) |
| HYALURONIC ACID | • Decrease of NFKB nuclear translocation (4h) |
| | • Decrease of cytoplasmic NFKB (24h) |
| HYALURONIC ACID +LPC-S01 | • Decrease of nuclear translocation (4h) |
| | • Decrease of cytoplasmic NFKB (24h) |

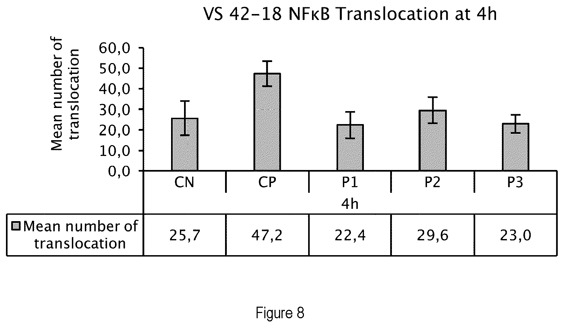

The results obtained at 4 h are as shown in Figure 8.

A significant efficacy in reducing the inflammation was observed for all the products at 4h.

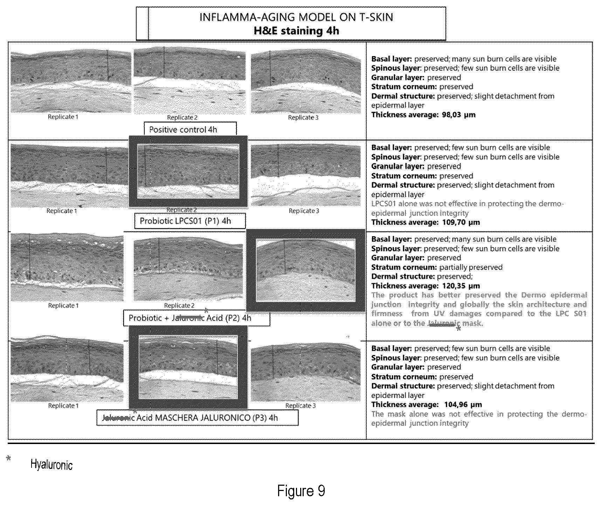

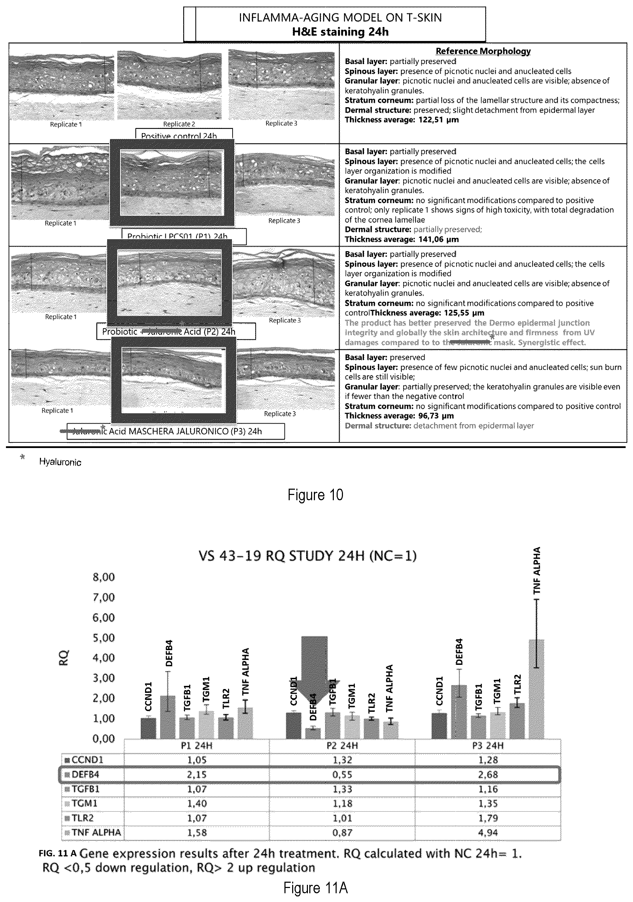

The mask has probably act as film forming agent protecting from the UV light. However, the tissue morphology in the sample treated with the P2 was significantly different at 4h and 24h as shown in Figures 9 and 10.

The product hyaluronic acid+LPC-S01 has better preserved the Dermo epidermal junction integrity and globally the skin architecture and firmness from UV damages compared to the Hyaluronic mask without the bacterial strain according to the invention, showing a clear synergistic effect.

### 3a.3 Summary of the results

The Probiotic LPC-S01 alone (P1) and the Probiotic LPC-S01 in combination with HA (P2) have been significantly active in reducing the effect of a biologically relevant UV dose (1 MED) in presence of a impaired skin barrier on NF-κB nuclear translocation and subsequent accumulation in the cytoplasm of the keratinocytes: they decreased first the nuclear translocation (early efficacy visible at 4h) and in a second time the cytoplasmic content of NF-κB (visible at 24h), indicating their delayed positive efficacy on inflammasome reduction.

Probiotic LPC-S01 (P1) has induced a further reduction of the cytoplasmic content of NF-κB at 4h.

In the specific exposure conditions adopted in this study (overnight treatment, gentle washing of residual, abrasion to reduce barrier function and to induce higher sensitivity to UV, exposure to UV) P1 and P2 have shown to act through an anti-inflammatory mechanism visible 4h after stress induction and still active after 24h.

Despite a similar anti-inflammatory efficacy, a synergistic effect was visible for P2 in the protection of the dermo-epidermal junction structure from UV damages. Moreover, a more efficient and long lasting (24h) efficacy of the probiotic is obtained when used in combination with the HA mask (P2).

### 3b. Probiotics + mask - inflammasome model - live and inactivated cells

An experimental model on T-skin (full thickness skin) based on inflammatory pathways induced by UVA + UVB (dose of 1 MED) has been applied to assess the efficacy of the probiotic strain LPC-S01 (viable and inactivated) when applied before the induction of the inflammasome stress.

The present study has been planned and conducted with 2 aims: to assess the efficacy of the Viable LPC-S01 itself or when introduced in a reference formulation ("Mask") after a short pre-treatment time (45 min) with final readout after 4h compared to long-term pre-treatment time (16 h overnight as in the previous study).
- to assess the efficacy of the Inactivated LPC-S01 either for short (45min) and long-term (16h overnight) pre-treatment time.

### 3b.1 Background

In the present study a VitroScreen "Full-thickness skin model" (T-skin) reproducing dermal and epidermal compartments has been used: this model has allowed the study of dermal extracellular matrix modification and epidermal differentiation with a multi-parametric approach in a stress condition induced by UV radiation.

### 3b.2 The study

A study has been conducted in order to assess the efficacy of *Lactobacillus paracasei* probiotic strain LPC-S01 itself or when introduced in a topical formulation ("Mask") compared to the formulation placebo (3 products): the protocol was based on inflammatory pathways induced by UVA + UVB (dose of 1 MED).

In this study the products were applied for an overnight pre-treatment time then removed and the T-skin was irradiated: the read out (NF-kB translocation and morphology) were quantified after 4h and 24h after irradiation.

The samples tested and the experimental conditions (products were applied directly on the surface of the 3D model) are reported in the following table.

**Table 9**

| TABLE I: TEST ITEMS | INTERNAL CODE | TREATMENT/ READ OUT (NF-kB) |
|---|---|---|
| Probiotic strain LPC-S01 | P1 | 45 min + 4h |
| Hyaluronic Acid Mask + LPC-S01 | P2 | |
| Hyaluronic Acid Mask - placebo | P3 | |
| Hyaluronic Acid Mask + inactivated LPC-S01 | P4 | |
| Inactivated Probiotic strain LPC-S01 | P5 | |
| Hyaluronic Acid Mask - placebo | P3-ON | Over night + 4h |
| Hyaluronic Acid Mask + inactivated LPC-S01 | P4-ON | |
| Inactivated Probiotic strain LPC-S01 | P5-ON | |

### Long-term 16h pre-treatment time protocol: comparison between viable and inactivated probiotic

In the table below, the semi-quantitative analysis of NF-κB nuclear translocation is presented for the 16 h (long term) pre-treatment time, comparing the present data with previously obtained (see previous study) to evaluate the effect of long-term treatment.

**Table 10**

| Tab. III NF-kb translocation (Mean±st.dev.) | | VS 75-18 | VS 42-18 |
|---|---|---|---|
| Negative Control | CN | 11,0 ± 2,1 | 25,7 ± 8,3 |
| Positive Control (Inflammasome) | CP | 18,8 ± 4,8 (+70,7%, p=0,01) | 47,2 ± 6,2(+83,7 p=0,01) |
| Treatment | | 16h overnight | 16h overnight |
| Probiotic LPC-S01 | P1 | -- | 22,4 ± 6,6 (-52,2% p=0,00) |
| Probiotic LPC-S01 + Mask | P2 | -- | 29,6 ± 6,3 (-37,3% p=0,00) |
| Mask placebo | P3 | 13,4 ± 1,3 (-28,4%, p=0,00) | 23,0 ± 4,4 (-51,3% p=0,00) |
| Inactivated LPC-S01 + Mask | P4 | 16,0 ± 3,2 (-14,8%) | - |
| Inactivated LPC-S01 | P5 | 18,9 ± 3,2 (+0,6%) | - |

- Inactivated LPC-S01 series (inactivated LPC-S01 itself or in the formulation) have not induced a significant reduction of NF-kB, which was measured after 16h pre-treatment whereas the viable series LPC-S01 (LPC-S01 itself or in the formulation) (ref. VS 42-18) were effective in restoring the level of nuclear NF-kB to negative control for all the test items with significant decrease of NF-kB translocation.
- Considering placebo mask, in the present study an efficacy was detected also after 16h pre-treatment (-28,4% p=0,00) as reported in the previous study.

In the following table the results of histo-morphological analysis are reported:

**Table 11**

| Tab. V Results analysis in terms of inflammasome pathway reduction and benefits for the skin | | | | |
|---|---|---|---|---|
| Read-out parameters | | NF-kB | | SBC |
| Prediction model in Inflammasome | | An increase of NF-kB translocation in cell nuclei is expected | | An increase of Sun Burn Cells is expected |
| Treatment | | 45 min | | |
| Probiotic LPC-S01 | P1 | +++ | | + |
| Probiotic LPC-S01 + Mask | P2 | +++ | | +++ |
| | | +++ | | |
| Mask placebo | P3 | - | | + |
| Inactivated LPC-S01 + Mask | P4 | + | | - |
| Inactivated LPC-S01 | P5 | - | | + |

| Treatment | | 16h (over night) | | |
|---|---|---|---|---|
| Mask | P3 ON | - | + | |
| Inactivated LPC-S01 + Mask | P4 ON | - | - | |
| Inactivated LPC-S01 | P5 ON | - | - | |

Scoring of the efficacy of treatments in reducing inflammasome (i.e. NF-kB positive nuclei and SBC). Legenda: (+) slight efficacy; (+++) marked efficacy; (-) no effects.

### 3b.3 Summary of the results

The biological relevance and reproducibility of inflammasome model (UV exposure at 1 MED, minimal erythemal dose) has been confirmed by an increase of NF-kB translocation in cell nucleus in irradiates samples in comparison with negative control.
- The relative increase of NF-kB translocation (percent difference) is comparable (+70,7%, p=0,01- VS 75-18) to the one quantified in previous study (+83,7, p=0,01- VS 42-18).
- Products containing the Viable probiotic LPC-S01 after short pre-treatment (45 min) showed a lower efficacy in reducing NF-kB translocation in comparison with long-term pre-treatment (16H).
- Inactivated probiotic LPC-S01 itself or when introduced in the formulation ("Mask") shows a low efficacy in reducing the UV induced damage (NF-kB translocation) either short and long term pre-treatments.
- The hyaluronic mask alone (mask placebo) has confirmed its action as physical shield with both short term (-27,2%, p=0,001) and long term (-28,4% p=0,00) pre-treatment.

### 4. Probiotic inactivated + Yaluage^{®} cream

The study is divided in three different phases:
1. Evaluation of the efficacy of the inactivated probiotic strain LPC-S01, alone or included in a cream formulation (Yaluage) on a complete 3D model reconstituted *in vitro,* including the dermis and epidermis (Full Thickness skin model), in terms of benefits for the skin homeostasis to explore its potential application and efficacy for skin care. (homeostatic model)
2. The previously studied dose will be tested for its effectiveness on the inflammasome model with pre-treatment (with respect to irradiation) of 45 minutes and 4 hours in 3 forms: a) as such b) in the formulation of Yaluage and c) Yaluage as such (control). (inflammasome model).
3. The previously studied dose will be tested for its effectiveness on the inflammasome model with application after 45 min and 4 hours after irradiation (post-treatment) in 3 forms: a) as such (bacterial probiotic strain LPC-S01) b) in the Yaluage formulation and c) Yaluage (inflammasome model).

The Yaluage cream is an anti-ageing face cream, comprising hyaluronic acid, collagen, vitamin E, Gardenia jasmoides (gardenia) stem cells, free and bond chemical filters and shea butter.

### 4a. Homeostatic model

The aim of the study was to investigate the inactivated bacterium strain (LPC-S01) skin tolerance profile after exposure at high concentrations and to assess its efficacy (alone or in the cream) in:
- enhancing skin autodefence by induction of anti-microbial peptides.
- stimulating keratinocytes innate immuno-response, epidermal renewal and differentiation.

### 4a.1 Background

In the present study a "Full-thickness skin model" developed by VitroScreen has been used. VitroScreen has specifically developed for cosmetic product testing an homeostasis model on "Full-thickness skin model" (T-skin) reproducing dermal and epidermal compartments: this model has the specificity to allow the study of dermal extracellular matrix modification and viable layer differentiation by a multi-parametric approach.

### 4a.2 The study

The efficacy of the inactivated probiotic strain LPC-S01 alone or included in a cream formulation (Yaluage) in terms of benefits for the skin homeostasis was evaluated on the T-Skin model.

The study has been conducted by using a high concentration of inactivated bacterium (10⁹ cells/tissue) in order to explore its potential application and efficacy for skin care.

Specifically, the products:
- inactivated LPC-S01 resuspended in saline solution (10⁹ cells/tissue) (univocal code=P1),
- the Yaluage cream (univocal code=P2),
- inactivated LPC-S01 resuspended in Yaluage cream (10⁹ cells/tissue), corresponding to 30% of the final formulation (univocal code=P3),
were applied directly on the surface of the 3D model for 24h and 48h.

The following parameters have been analyzed compared to untreated control:
- IL-1α release in the medium;
- Gene expression of key biomarkers of skin defence (DEFB4), innate immune response (TLR2, TNFα), epidermal differentiation and renewal (TGMS-1, CCND1, TGF-β1);
- Histological analysis by H&E.

Please find below the biological meaning of the markers above.

**Table 12**

| MECHANISM | BIOMARKER / PARAMETER |
|---|---|
| Skin tolerance and effect on skin homeostasis | Histomorphological analysis (H&E) IL-1α release in the medium (ELISA) |
| Boosting keratinocyte defense and innate response and bacterial associated inflammatory response | HBD2, TLR2, TNFα gene expression |
| Boosting skin renewal and differentiation | TGM1, CCND1, TGF-β1 gene expression |

The results are described below.

The study has been conducted by using a very high concentration of inactivated bacterium (10⁹ cells/tissue). Specifically, the tested products:
- inactivated LPC-S01 resuspended in saline solution (10⁹ cells/tissue) (labelled as=P1),
- the Yaluage cream (labelled as=P2),
- inactivated LPC-S01 resuspended in Yaluage cream (109 cells/tissue), corresponding to 30% of the final formulation (labelled as=P3),
were applied directly on the surface of the T-Skin model for 24h and 48h.

The Yaluage cream is an anti-ageing face cream, based on hyaluronic acid, collagen, vitamin E, Gardenia jasmoides (gardenia) stem cells, free and bond chemical filters and shea butter, presenting the following properties: Anti-ageing, prevents wrinkles and fine lines, UVA & UVB protection, smoothes the epidermal layer, moisturising, emollient, antioxidant.

The specific parameters and their biological meaning are described in the following table:

**Table 12b**

| MECHANISM | BIOMARKER / PARAMETER |
|---|---|
| Skin tolerance and effect on skin homeostasis | Histomorphological analysis (H&E) IL-1α release in the medium (ELISA) |
| Boosting keratinocyte defense and innate response and bacterial associated inflammatory response | HBD2, TLR2, TNFα gene expression |
| Boosting skin renewal and differentiation | TGM1, CCND1, TGF-β1 gene expression |

The main results obtained are reported below:

**Table 13**

| PRODUCTS | RESULTS |
|---|---|
| LPC-S01 inactivated (P1) | *•* Significant up-regulation of human defensin β2 at both time points indicating efficacy in triggering the host defences |
| | • Significant TNFα gene expression after 48h, indicating the induction of inflammatory response at the high doses applied on the tissue, probably due to the mutualistic interaction bacteria-skin |
| | *•* Metabolic activation inducing an accelerated differentiation process with a dynamic pattern from 24h to 48h, with a highly differentiated tissue. Induction of a more dense and compact extracellular matrix → this result may be confirmed with specific staining |
| YALUAGE (P2) | • Modulation of human defensin β2 after 48h, indicating the efficacy of the cream it-self in boosting the host defences in a long-term application period |
| | • Maintenance of tissue morphology after 24h, with detachment of DE junction and partial loss of integrity after 48h |
| YALUAGE + LPC-S01 inactivated (P3) | • Up-regulation of human defensin β2 at all the time-points, suggesting a gain of efficacy in the mixed formulation in boosting the host defences |
| | • TNFα up-regulation after 48h, indicating an accelerated differentiation process with a dynamic pattern from 24h to 48h, with a highly differentiated tissue and modification to stratum corneum. Induction of a more dense and compact extracellular matrix → this result may be confirmed with specific staining |
| | • Metabolic activation inducing an accelerated differentiated tissue and modification to stratum corneum. Induction of a more dense and compact extracellular matrix → this result may be confirmed with specific staining |

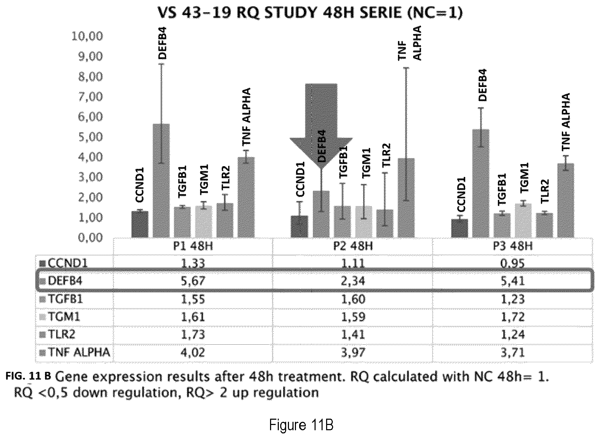

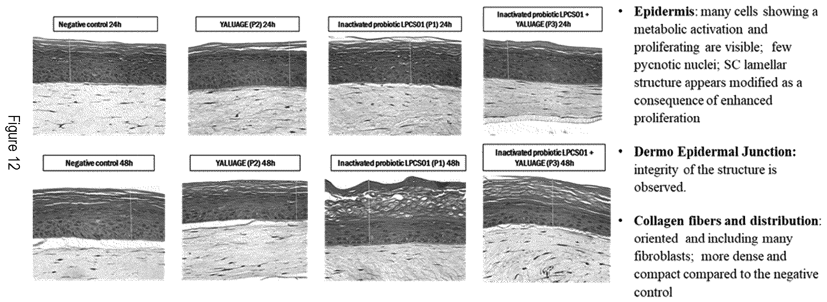

The results on the human defensin β are reported in Figure 11. While the effect of the combination on skin differentiation process is reported in Figure 12.

### 4a.3 Summary of the results

In this homeostatic model, the effects were observed with the inactivated LPC-S01 and Yaluage cream combination can be summarized as follows:
- Up-regulation of human beta-2 defensins at all times considered (24 and 48 hours), suggesting an increase in effectiveness by the aforementioned combination in increasing the host's defences.
- Metabolic activation that induces an accelerated process of differentiation with a dynamic pattern from 24h to 48h, with highly differentiated tissue and changes to the stratum corneum (fig. below).

Thus, the combination was well tolerated in the 3D skin model and was able to stimulate the body's defences and cell differentiation processes.

### 4b. Inflammasome model

The aim of the study was to investigate the inactivated bacterium LPC-S01 efficacy at two different doses (alone or in the cream) in modulating NF-kB activation and translocation to nucleus, when applied before or after the induction of the inflammasome stress.

In the present study a "Full-thickness skin model" developed by VitroScreen has been used. VitroScreen has specifically developed for cosmetic product testing an inflammasome model on "Full-thickness skin model" (T-skin) reproducing dermal and epidermal compartments: this model allows the study of dermal extracellular matrix modification and epidermal differentiation with a multi-parametric approach in a stress condition induced by UV radiation.

### 4b.2 The study

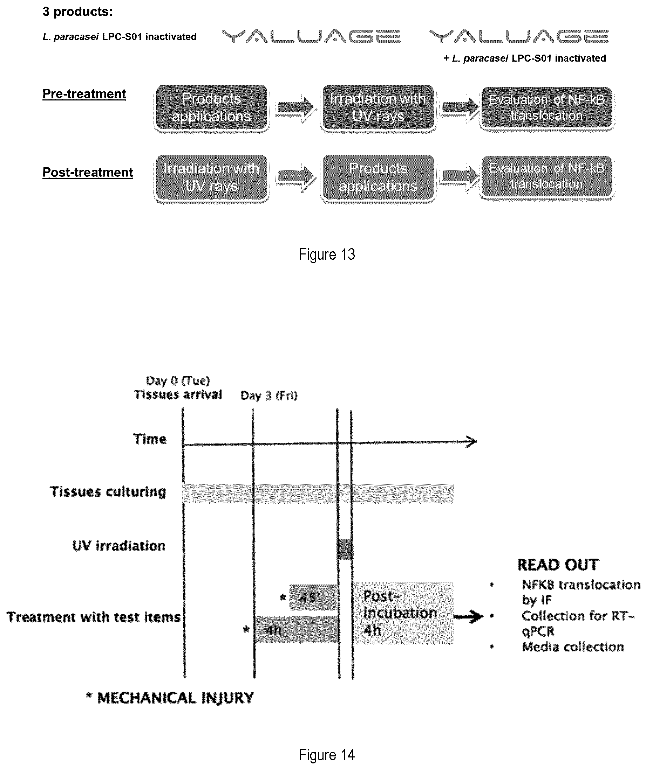

The inactivated probiotic strain LPC-S01, alone or included in a cream formulation (Yaluage), was evaluated by using two different concentrations of inactivated bacterium (10⁷ or 10⁹cells/tissue) in order to explore its potential application and efficacy on T-Skin inflammasome model according to 2 protocols, pre-treatment and post-treatment, as reported in Figures 13-15.

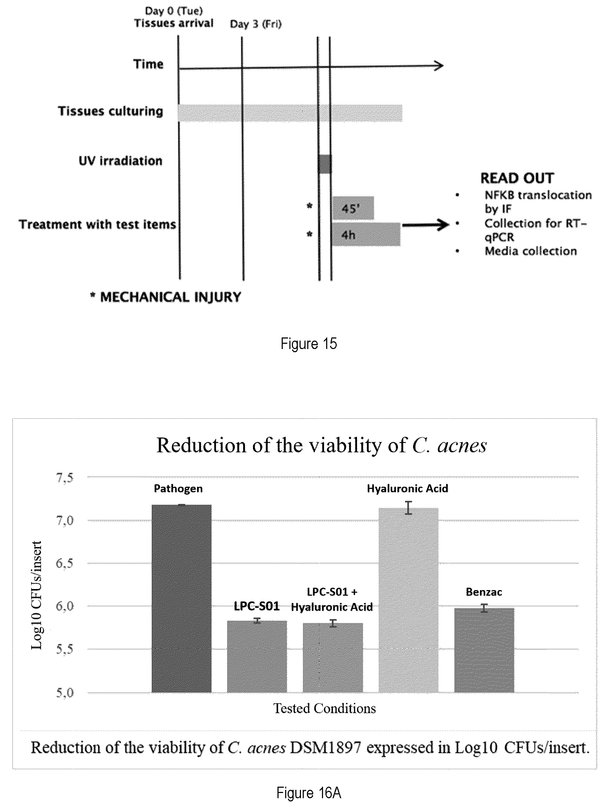

Specifically, the products have been tested on T-skin according to 2 protocols:
- inactivated LPC-S01 resuspended in saline solution (10⁹ cells/tissue) (labelled as=P1);
- the Yaluage cream (labelled as=P2);
- inactivated LPC-S01 resuspended in Yaluage cream at 2 concentrations (10⁹ and 10⁷ cells/tissue), corresponding to 30% and 0,03%, respectively, of the final formulation (labelled as=P3-10⁹ and P3-10⁷);
- Pre-treatment protocol: T-skin abraded through a mechanical stress on epidermal surface and pre-treated for 45 min and 4h with the test items, then subjected to UVA and UVB (1 MED) irradiation. After a 4h post-incubation the tissues were collected for the analysis (Figure 14);
- Post-treatment protocol: T -skin abraded through a mechanical stress on epidermal surface and subjected to UVA and UVB (1 MED) irradiation, then treated for 45min and 4h with the test items and immediately collected for the analysis (Figure 15).

The results are reported below.

**Table 14**

| **A. Pre-treatment: 45 min and 4h pre-treatment + 4h post UV incubation.** | | |
|---|---|---|
| | NF-kB positive nuclei | |
| | 45min+4h | 4h+4h |
| Negative Control (NC) | 0 | 0 |
| Positive Control (Inflammasome) | 7 | 5 |
| 10⁹ inactivated LPC-S01 (P1) | **3** | **1** |
| Yaluage cream (P2) | 0 | 0 |
| 10⁹ inactivated LPC-S01+ Yaluage cream (P3-10⁹) | **1** | **1** |
| 10⁷ inactivated LPC-S01+ Yaluage cream (P3-10⁷) | 5 | 2 |
| **LPC-S01 inactivated (P1)** | LPC-S01 alone counteracts NF-kB activation, indicating a preventive efficacy in protecting the skin from UV induced inflammatory stress. | |
| | If combined with the results obtained in the homeostatic model (VS 43-19), where a significant up-regulation of HBD2 was observed, these results indicate a significant protective and preventive efficacy of LPC-S01 with a mechanism based on enhancing the host innate response. | |
| **YALUAGE (P2)** | Yaluage completely inhibited NF-kB translocation: this mechanism is related to a physical protecting "shield" against UV rays. | |
| | This physical mechanism (preventing the damages caused by the inflammasome after short-term treatments) is confirmed by Yaluage results when applied on homeostatic conditions for longer applications (VS 43-19): in this conditions it has determined the activation of HBD2 but also toxicity after 48h. | |
| **YALUAGE + LPC-S01 inactivated (P3-10⁹ and P3-10⁷)** | The combination of LPC-S01 **P3-10⁹** and Yaluage confirmed the anti-inflammatory preventive efficacy as a synergy compared to single components. | |
| | The pre-treatment at 2 different concentration suggests a dose-response positive mechanism: the highest dose of bacterium is more efficient in decreasing NF-kB. | |
| | These results confirm the immunomodulatory properties of the probiotic strain both at physiological level (as observed in VS 43-19) and as preventive treatment against UV- induced inflammatory stress. At the same time, the synergy guarantees skin local tolerance and induction of host defense mechanisms that should be further demonstrated. | |

**Table 15**

| **B. Post-treatment: 45 min and 4h after UV irradiation.** | | |
|---|---|---|
| | NF-kB positive nuclei | |
| | 45min | 4h |
| Negative Control (NC) | 0 | 0 |
| Positive Control (Inflammasome) | 9 | 4 |
| 10⁹ inactivated LPC-S01 (P1) | 13 | **1** |
| Yaluage cream (P2) | 1 | 0 |
| 10⁹ inactivated LPC-S01+ Yaluage cream (P3-10⁹) | **3** | **1** |
| 10⁷ inactivated LPC-S01+ Yaluage cream (P3-10⁷) | 0 | 9 |
| **LPC-S01 inactivated (P1)** | LPC-S01 anti-inflammatory efficacy when applied after UV stress requires longer time (4h) to allow tissue recovery to homeostatic levels; this mechanism is often observed for probiotics with anti-inflammatory efficacy. | |
| | At longer times of exposure as observed in VS 43-19, the metabolic activation was observed after 48h, confirming that the optimal response requires longer exposures. | |
| **YALUAGE (P2)** | As observed in the pre-treatment serie, Yaluage cream is able to counteract NF-kB translocation on the inflamed skin by a physical mechanism. | |
| | These results further suggest that Yaluage has higher efficacy when applied on inflamed tissue rather than skin in homeostatic conditions. | |
| **YALUAGE +LPC-S01 inactivated (P3-10⁹ and P3-10⁷)** | When included in the Yaluage formulation, LPC-S01 has determined a more efficient and quick recovery to basal levels of NF-kB, in particular after the short treatment (when the inflammatory response is at its maximum level) suggesting that this synergy is effective in restoring tissue homeostasis. | |
| | These results confirm that the combination Yaluage and inactivated LPC-S01 have efficacy both in homeostatic conditions (VS 43-19) and on UV-induced inflammasome skin. | |

In the Tables above, the results corresponding to the efficacy of inactivated LPC-S01 in reducing the inflammasome pathway are reported **in bold.** The best synergies observed between the inactivated LPC-S01 and the Yaluage are **underlined and in bold.**

### 4b.3 Summary of the results

In this experimental model on T-skin based on inflammatory pathways induced by UVA + UVB the inactivated probiotic strain LPC-S01, alone or in the cream, when applied before or after the induction of the inflammasome stress, has shown as follows:
- *Pre-treatment:* the combination of LPC-S01 at concentration of 109 cells/tissue and the cream confirmed the anti-inflammatory preventive efficacy as a synergy compared to single components. The pre-treatment at 2 different concentration suggests a dose-response positive mechanism: the highest dose of bacterium is more efficient in decreasing NF-kB and, therefore, the inflammation.

These results confirm the immunomodulatory properties of the probiotic strain both at physiological level and as preventive treatment against UV-induced inflammatory stress. At the same time, the synergy guarantees skin local tolerance and induction of host defense mechanisms.
- *Post-treatment*: when included in the cream formulation, LPC-S01 has determined a more efficient and quick recovery to basal levels of NF-kB, in particular after the short treatment (when the inflammatory response is at its maximum level) suggesting that this synergy is effective in restoring tissue homeostasis. These results confirm that the combination of the cream and inactivated LPC-S01 have efficacy both in homeostatic conditions and on UV-induced inflammasome skin.

### 5. Probiotic + mask vs P. acnes

The aim of the study was to evaluate the ability of the probiotic *Lactobacillus paracasei* LPC-S01 strain, alone and/or in combination with hyaluronic acid, to counteract the adhesion of *Cutibacterium acnes (formerly Propionibacterium acnes)* to a full thickness *in vitro* skin model. In order to extend the assessment to all possible infection situations, a competition model, an exclusion model and a displacement model were assessed in the frame of this project, based on the adaptation of the method described by Coman et al. in 2015.

### 5. 1 The study

An *in vitro* model of infection of full thickness skin inserts with *C*. *acnes* was used to evaluate the possible impact of high molecular weight hyaluronic acid and/or probiotic strain *L. paracasei* LPC-S01 on the infection capacity of the pathogen.

The potential effect of probiotic strain and/or hyaluronic acid on the pathogen was assessed by 3 different *in vitro* models:
- pre-treatment of the full thickness skin with probiotic and/or hyaluronic acid (exclusion model),
- simultaneous treatment of the full thickness skin with probiotic and/or hyaluronic acid and pathogen (competition model),
- post-treatment of the full thickness skin, primarily infected with the pathogen, with probiotic and/or hyaluronic acid (displacement model).

Specifically, the tests were carried out considering different treatment conditions, listed below:
1) no treatment, to evaluate the effective and undisturbed adhesive capacity of *C*. *acnes* DSM 1897 in the three models of exclusion, competition and displacement;
2) preventive, concomitant or post-treatment with *L. paracasei* LPC-S01 for 24h;
3) preventive, concomitant or post-treatment with 0.5% hyaluronic acid for 24h;
4) preventive, concomitant or post-treatment with a homogeneous mixture of hyaluronic acid and *L. paracasei* LPC-S01 for 24h;
5) preventive, concomitant or post-treatment with benzoyl peroxide for 24h (Benzac 10%, positive control).

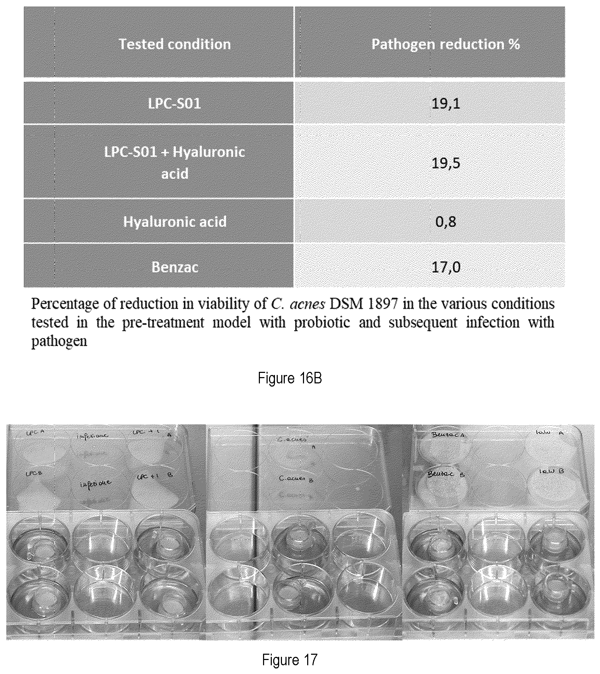

In Figure 16 are reported the results of the study.

### a) Exclusion assay

Based on the results reported in Figure 16, it is possible to state that the treatments carried out with Benzac 10%, LPC-S01 and the combination of LPC-S01 in the presence of 0.5% of hyaluronic acid reduce the vital charge of *C*. *acnes* by about 1.0-1.4 Log10, corresponding to about 20% reduction in pathogen viability. However, the treatment with 0.5% hyaluronic acid alone does not seem able to reduce the viability of the pathogen in any measure.

It should be emphasized that *L. paracasei* LPC-S01 strain shows affinity for the 3D Skin Full Thickness model. *L. paracasei* LPC-S01 strain is in fact able to actively multiply during incubation with the 3D Skin Full Thickness model, increasing its vital charge by just over half a logarithm in the pre-treated inserts.

The reduction of the pathogen *C*. *acnes* DSM 1897 following pre-treatment with the probiotic strain *L. paracasei* LPC-S01 could therefore be attributable to the acidification of the culture medium due to the active proliferation of the probiotic.

The slight deterioration observed in the concerned inserts at the end of the incubation period (a yellow change of the culture medium of the inserts) could also be attributed to the metabolic activity of the probiotic (Figure 17).

### b) Competition assay

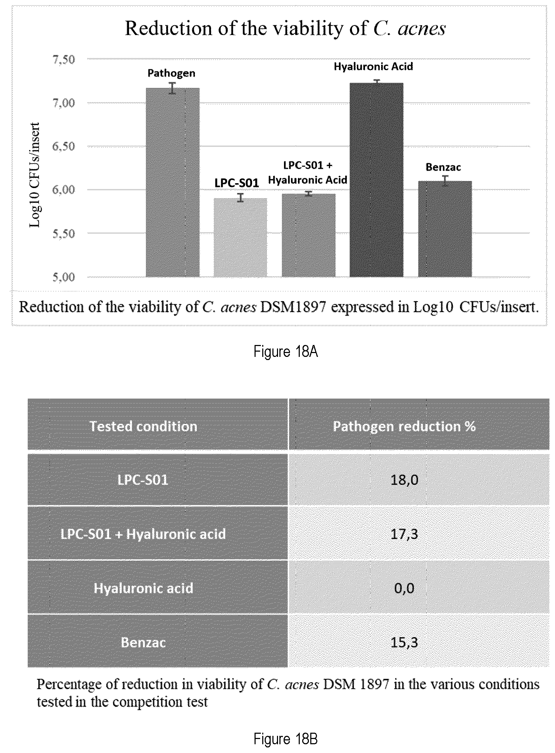

As reported in Figure 18, it was confirmed that the treatments carried out with Benzac 10%, LPC-S01 and the combination of LPC-S01 in the presence of 0.5% of hyaluronic acid reduce the vital charge of C. *acnes* DSM 1897 by 1.0-1.3 Log10 CFUs. The treatment with 0.5% hyaluronic acid alone does not appear to reduce the viability of the pathogen.

It should be noted that the *L. paracasei* LPC-S01 strain showed a very low affinity for the 3D Skin Full Thickness model during the incubation time. The count of the *L. paracasei* LPC-S01 strain showed a very slight increase, from 8.0 Log10 CFUs to 8.2 Log10 CFUs following 24h of incubation with the inserts. Consequently, for the competition model, the reduction of the pathogen C. *acnes* DSM 1897, following the simultaneous presence of the probiotic strain *L. paracasei* LPC-S01, is not clearly attributable to the proliferation of the probiotic, and therefore a different mechanism of action could be postulated.

### c) Displacement assay

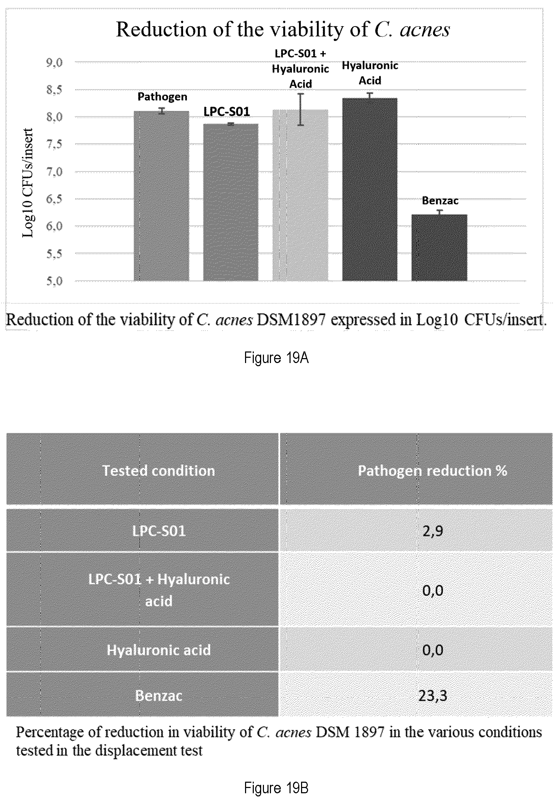

As shown in Figure 19, only the treatment carried out with Benzac 10% was able to reduce the growth of the pathogen by about two logarithms (about 23%).

All other post-infection treatments of *C*. *acnes* DSM 1897 do not appear to reduce the viability of the pathogen.

It should be noted that *L. paracasei* LPC-S01 strain was not able to actively multiply during the 24h displacement test. The count of the *L. paracasei* LPC-S01 strain was in fact showing a decrease following the 24h of incubation with the inserts. Consequently, for the displacement model, the slight reduction of the pathogen *C*. *acnes* DSM 1897 following the simultaneous presence of the probiotic strain *L. paracasei* LPC-S01 is not clearly attributable to the proliferation of the probiotic.

### 5.2 Summary of the tests

All the *in-vitro* tests have demonstrated the effectiveness of the 10% Benzac positive control in the containment of the infection by *C*. *acnes,* with % reduction of the vitality of the pathogen population from 15% to 23%. As shown by the displacement test, once the *C*. *acnes* DSM 1897 infection has started in the inserts, only the medical device has been proven to be able to effectively contain its replication.

The exclusion and competition tests showed that the treatments carried out with LPC-S01 and the combination of LPC-S01 + 0.5% of hyaluronic acid reduce the infection of *C*. *acnes* DSM 1897 by about 18%-19%. The probiotic effectiveness can be reasonably attributed, in the *in vitro* exclusion model, to the ability of the strain to proliferate in the culture medium of the insert, causing a slight acidification of the medium, while in the competitive model, with a shorter incubation time, this effect was not observed and consequently the significant pathogen inhibition by probiotic must be attributed to another mechanism.

The results of the displacement model showed a very slight positive effect of the probiotic (3% reduction in the viability of the population of the pathogen), albeit much lower than that observed for the 2 previously considered models.

The data obtained suggest that 0.5% hyaluronic acid does not produce any antagonistic effect towards the probiotic strain LPC-S01, which, even in combination with hyaluronic acid, maintains substantially unchanged its ability to counteract *C*. *acnes* DSM 1897.

### EXPERIMENTAL PART

### Aim of the study

The aim of the study was to evaluate the efficacy of the product "Infinite Skin Microbiome Serum" after a 14 and 28 days treatment through a clinical evaluation, an instrumental measurement of the skin hydration and a self-perception questionnaire.

### Product and How to use

An embodiment of the present invention is called "Infinite Skin Microbiome Serum" and it comprises: AQUA PROPANEDIOL, SODIUM HYALORONATE, PHENOXYETHANOL, LACTOBACILLUS FERMENT, MALTODEXTRIN, 1,2 HEXANEDIOL, CAPRYLYL GLYCOL, SODIUM ANISATE, HYDROLYZED HYALURONIC. Volunteers were asked to apply the cosmetic product as follows:

### Product activation:

- Remove the tab with a tear,
- Compress the plunger with the palm of your hand (easier if done on a hard, flat surface),
- Shake the bottle for 5-10 seconds to completely disperse the powder in the liquid.

### Storage:

- Product to be kept in the refrigerator after reactivation and after each application.

### How to use:

- The product must be applied in the evening and left to act at night (as last step in the nightly routine),
- Shake the bottle for 2-3 seconds before each application,
- Take 2 pipettes of serum, place the serum in the palm of your hand and apply it on the face and neck; let it dry (the product will dry in a few minutes),
- Wash your face in the morning.

### Objectives:

Evaluate the treatment efficacy after 14 and 28 weeks of usage in terms of:
- Changes in skin hydration through an instrumental evaluation made with MoistureMeter SC (Delfin).
- Verify the variations in terms of extension of the facial area involved by acne, variations in the number of pimples present, the red areas (erythema) and skin dryness of each volunteer's skin made through the dermatologist clinical assessment.
- Evaluate the efficacy perceived by the volunteers, through their assessment about the treatment after 14 and 28 days of usage and the presence or absence of side effects.
- Document with a digital camera an image of the skin condition before and after treatment.

### Target:

30 volunteers; male and female. Age: between 18 and 40 years old. Healthy subjects, without skin diseases, they do not have allergies and/or intolerance to cosmetics or drugs. Characteristics: subjects with acne-prone skin and with the presence of acne from mild to moderate entity.

### Test subjects:

The volunteers participating in the study were selected in the area of Modena from a panel of healthy subjects according to the following inclusion/non-inclusion criteria.

### Inclusion Criteria:

Age between 18 and 40 years old. Subjects with acne-prone skin and with the presence of acne from mild to moderate entity. Healthy subjects, without skin diseases or allergy/intolerance to cosmetics or drugs. Subjects who agree to follow the study procedures and respect the control steps.

### Exclusion Criteria:

Subjects under topical or systemic treatment with any drug that may influence the outcome of the efficacy study. Subjects affected by skin diseases. Pregnant or lactating women. Subjects with intolerance to drugs and/or cosmetics.

### Limitations:

For the entire duration of the study, volunteers were asked not to apply on the analysed skin area cosmetic products different than the one under study.

### Drop-out:

There was 1 case of drop-out.

Before the beginning of the test the investigator delivers to the volunteers the tested product and the informative form. Each subject at the beginning of the test, read and countersigned the informed consent to take part to the study. At time 0 and after 14 days, 28 days of treatment, were carried out the following evaluations:
- Instrumental measurements with the MoistureMeter SC (Delfin) to evaluate the surface hydration of the skin in a defined area of the face.
- Clinical evaluation made by dermatologist regarding the facial area interested by acne, the number of pimples, skin redness (erythema) and skin dryness.
- Documentation with digital camera an image from the condition of the skin.

After 14 days, 28 days of treatment and after 30 days from the end of the study, volunteers were asked to assess the treatment, in terms of perceived efficacy and side effects, answering a questionnaire.

### SURFACE HYDRATION EVALUATION

Instrumental measurements were carried out in a defined area of the face treated with the product in test. For each volunteer, were carried out 3 repetitions at each step with the following probe:
MoistureMeter SC (Delfin). The probe measures the hydration levels of the skin surface at the level of the stratum corneum.

CLINICAL EVALUATION: Clinical assessments were taken by the dermatologist according to the clinical scores showed in the tables below.

Clinical classification of the extension of the facial area involved:
No area involved 0 *
<25% of the face 1
Between 25% and 50% of the face 2
> 50% of the face 3

### Clinical classification of erythema:

No evidence of erythema 0 *
Barely noticeable erythema 0,5
Slight erythema 1
Moderate redness 2
Strong uniform redness 3
Fiery redness 4

* At T0 the value 0 is considered as an exclusion criteria.

### Clinical classification of skin dryness:

Absent dryness 0
Very slight dryness 0,5
Slight dryness 1
Moderate dryness 2
Severe dryness 3
Severe dryness with visible peeling 4

### 1. Instrumental results:

### Skin surface hydration (MoistureMeter SC - Delfin).

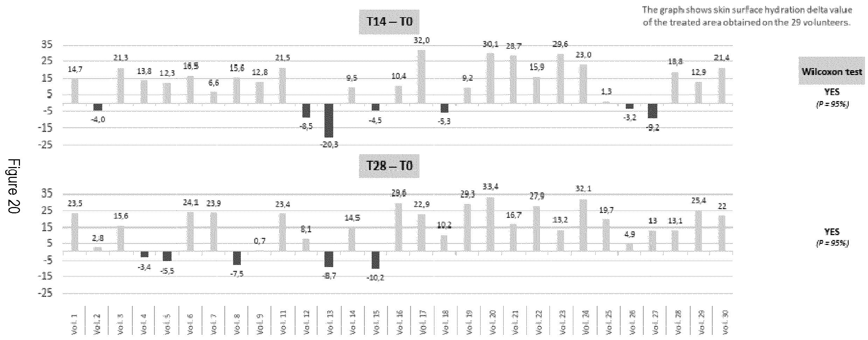

The graphs reported in Figure 20 show the delta of the instrumental evaluation data recorded for each volunteer between T0 and 14 days and 28 days of treatment with the cosmetic product. The Wilcoxon test for paired data was carried out on the data obtained. This test, for non-parametric data, allows to establish if there is a statistically significant efficacy of the treatment (P = 95%).

The graph on Figure 20 shows skin surface hydration value of the treated area obtained on the 29 volunteers.

In Figure 20 are shown, for each volunteer, the differences between the mean value at T 14-days and T 28-days compared to T0. After 14 and 28 days of the treatment it is observed a statistically significant improvement in skin surface hydration.

### 2. Clinical results:

Clinical evaluation of the extension of the facial area involved.

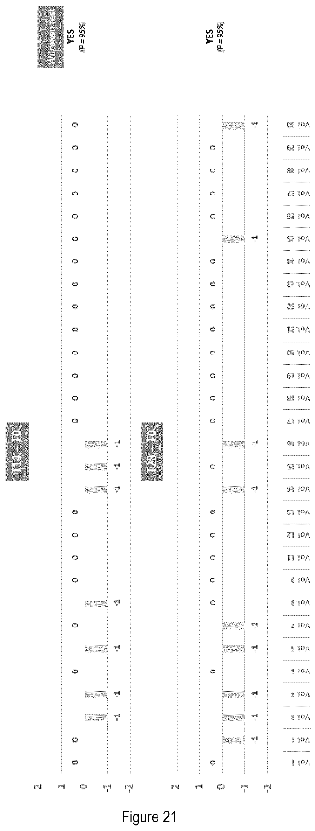

The graphs on Figure 21 show the delta of the clinical evaluation data recorded by the dermatologist for each volunteer, between T0 and 14 days and 28 days of treatment with the cosmetic product. The Wilcoxon test for paired data was carried out on the data obtained. This test, for non-parametric data, allows to establish if there is a statistically significant efficacy of the treatment (P = 95%).

The graph on Figure 21 (Clinical evaluation of the extension of the facial area involved) shows delta values obtained on the 29 volunteers.

### 3. Clinical results:

### Erythema.

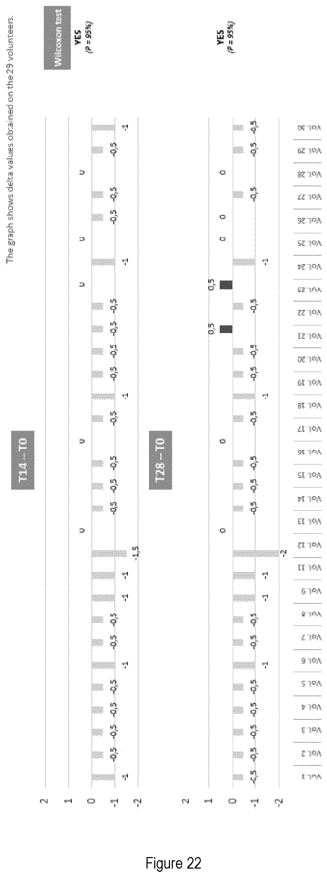

The graph on Figure 22 shows delta values obtained on the 29 volunteers.

Clinically, after 14-days and 28-days of treatment, a statistically significant decrease is recorded regarding the erythema.

### 4. Clinical results:

### Number of papules and/or pustules.

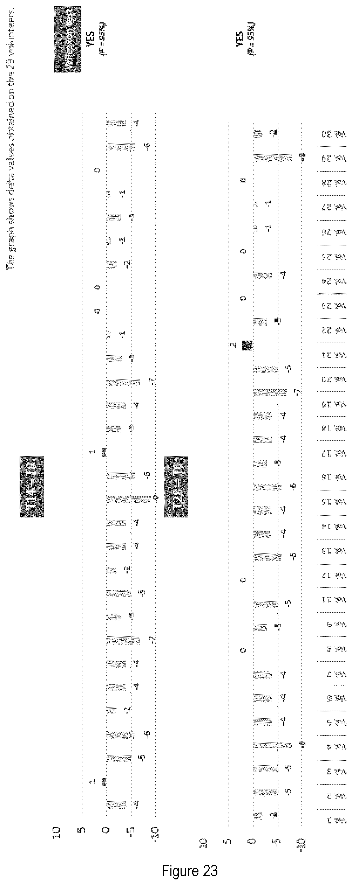

The graph on Figure 23 shows delta values obtained on the 29 volunteers.

Clinically, after 14-days and 28-days of treatment, a statistically significant decrease is recorded regarding the number of papules and/or pustules.

### 5. Clinical results:

### Skin dryness.

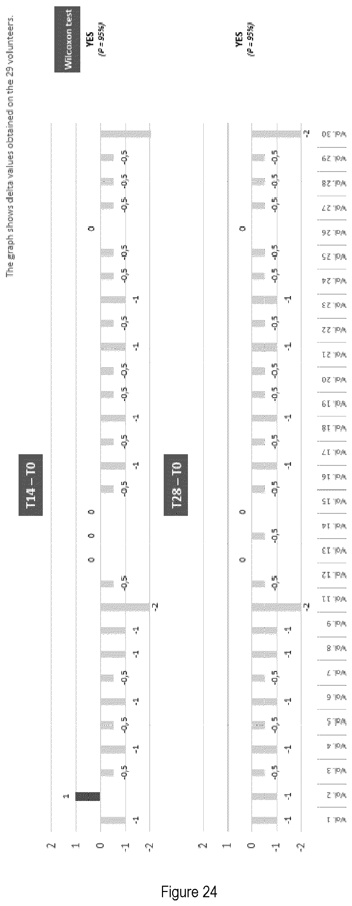

The graph on Figure 24 shows delta values obtained on the 29 volunteers.

Clinically, after 14-days and 28-days of treatment, a statistically significant decrease is recorded regarding the skin dryness.

### 6. Self-perception results:

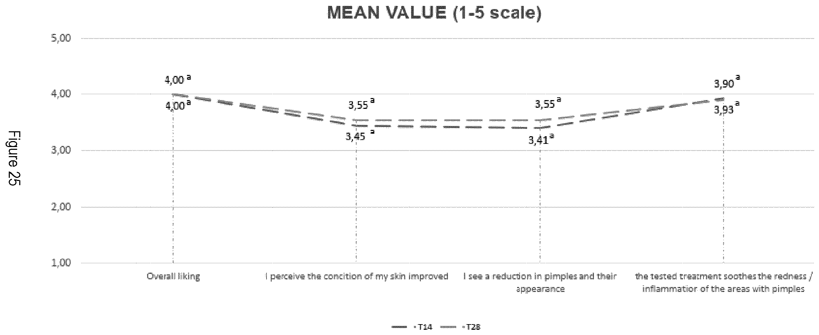

based on 29 cases per sample after 14-days versus 28-days treatment is shown on Figure 25.

Volunteers evaluations were subjected to variance analysis (P=95%) and to the Least Significant Difference test (LSD P=95%). From a graphical point of view, the results of LSD-Test are marked in the graph by the application of one or more letters near the mean values: for each item, two steps can be considered statistically distinguishable, with a probability P≥95%, if they are not marked with the same letter.

### Conclusions

### (i) Instrumental and clinical conclusions

Both after 14 and 28 days of treatment, on the panel of volunteers involved, the instrumental measurements carried out with the MoistureMeter SC (Delfin) showed a statistically significant increase of skin hydration. The clinical evaluation performed by the Dermatologist showed a statistically significant reduction in the extension of the facial area involved by acne, a statistically significant decrease in skin redness, in the number of papules and/or pustules and skin dryness.

### (ii) Self-perception conclusions

Overall, the treatment was appreciated by the volunteers both after 14 and 28 days of treatment. The volunteers quite agree in perceiving an improvement in the skin regarding the redness/inflammation of the area with pimples. The degree of agreement for the statements "I perceive the condition of my skin improved" and "I see a reduction in pimples and their appearance" is good. The treatment was also well tolerated by all the subjects participating in the study (no adverse skin reactions were monitored during the whole test period of 28 days).

### EFFECTS OF Lacticaseibacillus paracasei m.biome LIVESKIN88 (DSM 33788) + HYALURONIC ACID (HA) DRY CAP MASK ON WOUND HEALING, NFkB EXPRESSION AND SKIN AGING

### Aim of the study

The aim of this project is to explore the efficacy of hyaluronic acid mask (HA) in presence of probiotic strain *Lacticaseibacillus paracasei* m.biome *LIVESKIN88* (DSM 33788) as skin care product. In particular, the synergistic effect of these two ingredients will be assessed through the evaluation of following points:
- Wound healing
- Adhesion/colonization property
- NFkB activation
- Antioxidant property
- Anti-pathogenic property

### Material and method

### HaCaT adhesion assay

The adhesion of Liveskin88 to HaCaT cell layer was assessed as described (Guglielmetti, 2008) with light changes. In brief, HaCaT cells were grown in Dulbecco's Modified Eagle's Medium (MEM) supplemented with 10% (v/v) heat-inactivated fetal calf serum, 100 U ml-1 penicillin, 100 mg ml-1 streptomycin, 0.1 mM non-essential amino acids, 2 mM L-glutamine and incubated at 37° C in an atmosphere of 95% air and 5% carbon dioxide. For adhesion experiments, 2 x 10⁵ cells were incubated for 24 h. Different dilution of complete mask (1 to 2 and 1 to 10) were incubated with a monolayer of HaCaT cells for 1 h at 37° C. Monolayers were washed three times with phosphate-buffered saline pH 7.3 (PBS) to release unbound bacteria and incubated with 3 ml of methanol for 8 min at room temperature to fix cells. Afterwards, cells were stained with 3 ml of Giemsa stain solution (1:20; Carlo Erba, Milano, Italy) and left 30 min at room temperature in the dark. Finally, monolayers were washed three times with PBS, dried in an incubator for 1 h, and examined microscopically (magnification, 400×) under oil immersion.

### In vitro HaCaT wound healing

Wound healing in response to HA mask stimulation was assessed by a scratch assay on a HaCaT cell monolayer. Briefly, 5 × 10⁵ HaCaT cells were seeded into each well of culture inserts and incubated at 37 °C in a humidified atmosphere with 5% CO₂. After 24 h, the culture inserts were gently removed using sterile tweezers, and a scratch was made in the monolayers. Photographs of the wounded area were taken immediately before stimulation (0 h time point) and after the choose incubation time to monitor the closure of the wounded area. The percentage of wound closure was calculated as (Area initial - Area final)/Area initial × 100.

### NF-κB activation assay

The activation nuclear factor κB (NF-κB) was studied by means of a recombinant HaCaT cell line stably transfected with vector pNiFty2-Seap (InvivoGen, Labogen, Rho, Italy). In brief, recombinant Caco-2 monolayers (approximately 5 × 10⁵ cells/well), cultivated in the presence of 50 µg ml-1 zeocin, were washed with 0.1 M Tris-HCl buffer (pH 8.0) and then incubated with 5 × 10⁷ cells of *Lacticaseibacillus paracasei* m.biome *LIVESKIN88* (DSM 33788) product suspended in fresh DMEM containing 100 mM HEPES (pH 7.4), resulting in a MOI of approximately 100. In order to evaluate the immunomodulation property of each product component, HA and complete mask (HA + *Lacticaseibacillus paracasei* m.biome *LIVESKIN88* (DSM 33788)) was diluted 1 to 20 in PBS and added to HaCaT cells monolayer.

Stimulation was conducted by adding 20 ng ml-1 of TNF-a. After incubation at 37° C for 4 h SEAP in the supernatant was revealed using the Quanti-Blue reagent (Invivogen) according to the manufacturer's protocol and quantified at 655 nm OD. All measurements were performed using a microplate reader (Multiskan SkyHigh, Thermo Fisher Scientific). Two independent experiments were conducted in triplicate for each condition.

### (2,2-Diphenyl-1-Picrylhydrazyl) Free Radical Scavenging Activity

The free radical scavenging activity of *Lacticaseibacillus paracasei* m.biome *LIVESKIN88* (DSM 33788) was examined by DPPH assay with some modifications. Briefly, the probiotic suspension at the concentrations of 1 x 10⁹ cells/mL was added to 1 mM of DPPH solution in a 96-well plate. The mixture was incubated for 30 min at room temperature and protected from light. When DPPH receives a hydrogen atom from an antioxidant sample, its color changes from violet to light yellow. The absorbance was measured at 517 nm with a microplate reader. Ascorbic acid at a concentration of 1 µg/mL and was used as a positive control.

### ROS Production in HaCaT Cells

Intracellular ROS concentrations were assessed using a dichloro-dihydro-fluorescein diacetate (DCFH-DA) assay. The HaCaT cells were seeded at 3.0 × 104 cells/well in 96-well plates for 24 h. After incubation, the medium was removed. The cells were washed with an excess of PBS, pre-treated with 10 µmol/L of DCFH-DA in serum-free media at 37 °C for 45 min The cells were then washed with an excess of PBS and treated with probiotic suspension and H₂O₂ at the concentration of 1500 µmol/L in serum-free medium at 37 ∘C. Finally, the ROS levels were measured using the microplate reader at the excitation and emission wavelength of 485 and 530 nm, after 3 h.

### Antimicrobial assay

Antimicrobial activity was determined by the agar-spot test method. Briefly, each isolate of LAB was spotted on MRS agar and incubated under anaerobic conditions at 37°C for 72 h. Then, the plate was overlaid with 6 ml of soft MRS agar (0,9% agar) seeded with 1 ml overnight culture of *S*. *aureus* strain. The activity of *Lacticaseibacillus paracasei* m.biome *LIVESKIN88* (DSM 33788) was studied in comparison with the activity of the following strains: *L. rhamnosus GG, L. acidophilus* LA5 and *L. paracasei* SHIROTA. Chlorexidin was used as control.

### Results and discussion

### Effect of Lacticaseibacillus paracasei m.biome LIVESKIN88 (DSM 33788) mask on wound healing

Skin damage can be caused by a variety of different reasons such as trauma (including cuts, abrasions, chemical burns, fire burns, cold, heat, radiation, surgery), or as a consequence of underlying illnesses such as diabetes. The most effective wound management strategy is to prevent infections, promote healing, and prevent excess scarring.

Conventional mono-layered cultures are easy, cheap, and relatively fast in displaying results. Monolayers of cells, e.g., human epidermal keratinocytes are commonly disrupted using a sterile wounding instrument (scratch assay) providing the general draw back in understanding the mechanisms governing wound healing. One of most use cell line model for wound healing study is HaCaT, a spontaneously mutated keratinocyte cell line from immortalized adult skin.

Several pieces of evidence support HA as a key component of the extracellular matrix involved in different biological process, such as proliferation and migration. To address the hypothesis of synergic effect between HA and *Lacticaseibacillus paracasei* m.biome *LIVESKIN88* (DSM 33788) cosmetic product in the regenerative process of human skin, we tested their effects on proliferation *in vitro* using HaCaT cells. There is also some published literature on the use of models to simulate wound healing, but there is still no published literature on the use of probiotics with them.

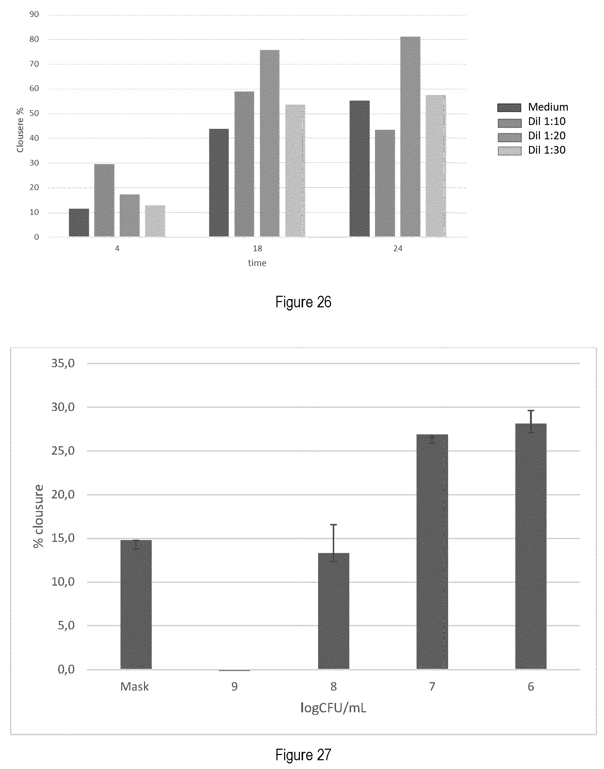

Firstly, we measure the ability to promote the wound healing of complete mask (*Lacticaseibacillus paracasei* m.biome *LIVESKIN88* (DSM 33788) freeze dry powder (8 x 10⁹ CFU/g). To do this, different dilution of complete product was used both to assess the toxicity of excipients and to evaluate the ability to promote the tissue regeneration after mechanical stress (Figure 26).

The results show that the application of complete mask (*HA+Lacticaseibacillus paracasei* m.biome *LIVESKIN88* (DSM 33788)) promotes the wound healing by scratch wounding confluent monolayer of HaCaT cells. Despite of the dilution 1 to 10 promotes the faster monolayer regeneration, the decrease in % of closure after 24 hours shows that it is toxic for the cells proliferation/migration. The best results, in term of % closure of HaCaT monolayer, were obtained with the dilution 1 to 20 with the slower but higher tissue regeneration. Moreover, this dilution mimics the real exposure of facial mask on face, considering seven applications of product for each bottle.

To investigate if the effect of regeneration is duo to at bacterial cell or HA effect, we performed some experiments using 1 to 20 HA mask alone and with different bacterial concentration of *Lacticaseibacillus paracasei* m.biome *LIVESKIN88* (DSM 33788).

To investigate the effect of probiotic addiction in the HA mask, the wound healing promotion after 4h was monitored using different concentration of *Lacticaseibacillus paracasei* m.biome *LIVESKIN88* (DSM 33788) resuspended in mask dilute 1 to 20.

As shown in Figure 27, no or lower tissue wound closure was obtained using high concentration of cells (9 and 8 logCFU/mL) resuspended in 1 to 20 mask. Probably this is an effect of low pH (between 4.5 and 5) of the tested suspension cause by high amount of freeze-dry *Lacticaseibacillus paracasei* m.biome *LIVESKIN88* (DSM 33788) dissolved mask excipients.

Good results were obtained with the lower concentration (7 and 6 logCFU/mL). In particular these data show a synergic effect given by the combination of HA mask and *Lacticaseibacillus paracasei* m.biome *LIVESKIN88* (DSM 33788). Notably, that 7 logCFU/mL correspond at the bacterial cell concentration of complete mask diluted 1 to 20. No significative difference was detected between the last two dilution

### Lacticaseibacillus paracasei m.biome LIVESKIN88 (DSM 33788) adhesion ability to HaCaT cell

Adhesion ability to the host is a classical selection criterion for potential probiotic bacteria that could result in a transient colonisation that would help to promote immunomodulatory effects, as well as stimulate skin barrier and metabolic functions. It is well known that probiotic bacteria have a potential protective role against pathogens through different mechanisms including production of antimicrobial compounds, reduction of pathogenic bacterial adhesion and competition for host cell binding sites.

Competitive exclusion, the competition between microorganisms for nutrients and binding sites and the production of antimicrobial substances, resulting in pathogen inactivation, is an important function of the normal healthy skin microbiota. Probiotics have been shown to have similar effects and adhesion to the skin may prevent the binding of some pathogenic organisms,

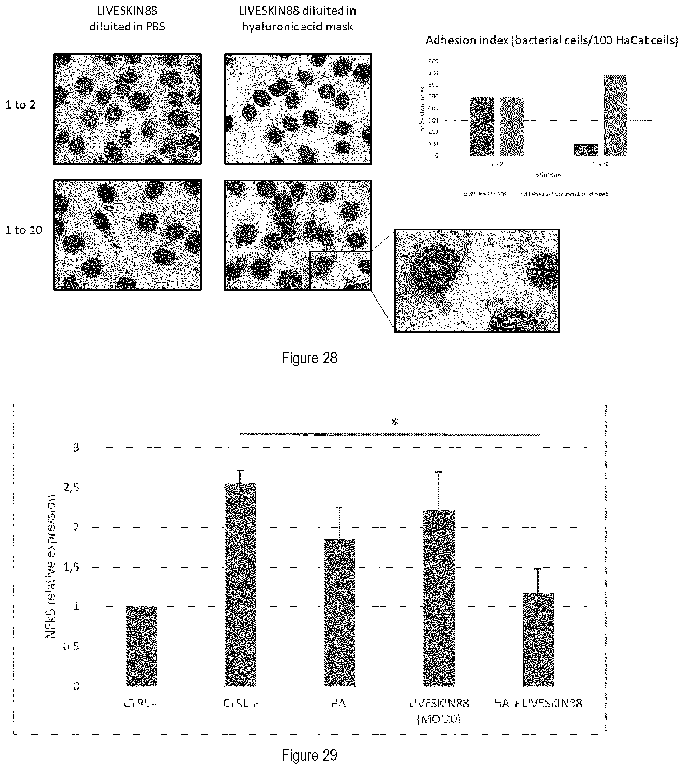

To evaluate the "boosting effect" of HA in adhesion property of *Lacticaseibacillus paracasei* m.biome *LIVESKIN88* (DSM 33788), we evaluated the adhesive property of *Lacticaseibacillus paracasei* m.biome *LIVESKIN88* (DSM 33788) on HaCaT cells in presence and in absence of HA mask (Figure 28).

The results showed that the resuspension of *Lacticaseibacillus paracasei* m.biome *LIVESKIN88* (DSM 33788) in HA mask guarantees an improvement of the adhesive property of bacterial cells. In fact, the adhesion index obtained with the bacterial cells resuspended in presence of HA, is higher compared the value obtained with the bacterial cells resuspended in PBS. Probably the improvement is duo to a mechanical effect of HA that create a layer of HaCaT skin model that improve a stability of adhesion of *Lacticaseibacillus paracasei* m.biome *LIVESKIN88* (DSM 33788) cells.

### Lacticaseibacillus paracasei m.biome LIVESKIN88 (DSM 33788) mask modulates NF-κB activation in HaCaT cells under inflammatory stimulation

NF-κB comprises a family of inducible transcription factors that are important regulators of the host immune and inflammatory responses. Because NF-κB activates the transcription of several proinflammatory cytokines, it is tightly coupled to inflammatory processes in the skin. The skin pathology includes epidermal hyperplasia, hyper-keratosis, parakeratosis, loss of the granular layer, T-cell infiltration, and the formation of micro-abscesses. These features are considered to be the hallmarks of the human inflammatory skin disorder, psoriasis, where it is well established that T cells play a central role.

The anti-inflammatory properties of the *Lacticaseibacillus paracasei* m.biome *LIVESKIN88* (DSM 33788) resuspended in HA product was assessed using the HaCaT/NF-κB reporter system. The experiments were carried out stimulating for 4 h the recombinant HaCaT cell layer at baseline and in presence of a proinflammatory stimulation with TNF-a.

The addition of TNF-a caused approximately doubled the activation levels of NF-κB (Figure 29). Notably, the presence of the *Lacticaseibacillus paracasei* m.biome *LIVESKIN88* (DSM 33788) or HA in both cases guarantees a reduction of NF-κB activation compared with inflamed condition (respectively 27% and 13% less). Strong and statistical significative effect was obtained using both components together suggesting an additive or synergistic action between *Lacticaseibacillus paracasei* m.biome *LIVESKIN88* (DSM 33788) and HA.

### Effect of Lacticaseibacillus paracasei m.biome LIVESKIN88 (DSM 33788) on ROS Production in HaCaT Cells under Oxidative Stress

The term reactive oxygen species (ROS) refers mainly to free radicals derived from molecular oxygen, and a few other chemically reactive molecules, which originate during the gradual reduction of molecular oxygen. Probiotic strains have been reported to scavenge hydroxyl radicals and superoxide anions and produce antioxidants. The most widely studied strains are currently *Bifidobacterium* and *Lactobacillus.*

DPPH assay is based on the capacity of the sample to scavenge DPPH radical. In this work, different probiotic suspensions (*Lacticaseibacillus paracasei* m.biome *LIVESKIN88* (DSM 33788), *L. paracasei* DG and *L. paracasei* SHIROTA) was used to evaluate ROS scavenging activity compared to ascorbic acid used as the positive control.

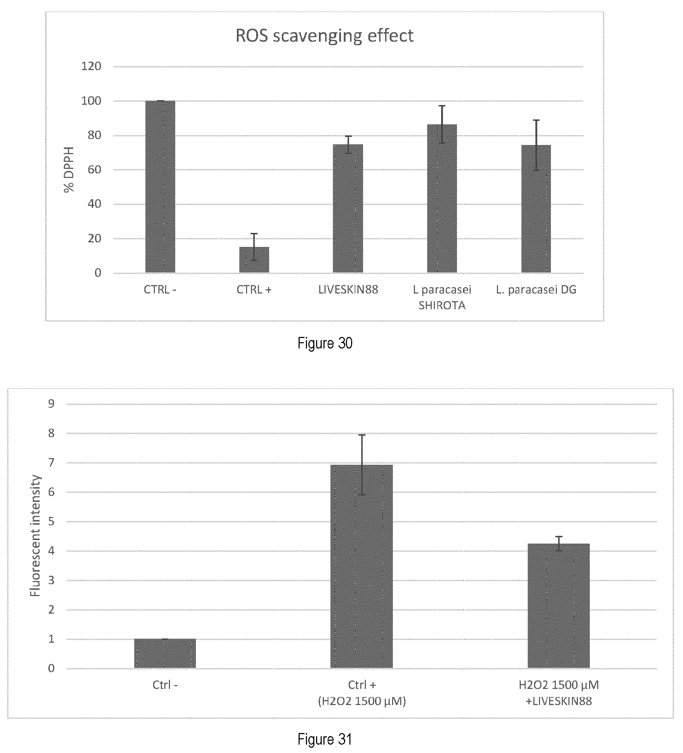

*Lacticaseibacillus paracasei* m.biome *LIVESKIN88* (DSM 33788) and *L. paracasei* DG exhibited the best performance in terms of scavenging effect. These results suggest that *Lacticaseibacillus paracasei* m.biome *LIVESKIN88* (DSM 33788) has a strong radical scavenger effect that could contribute to reduce the skin oxidative stress condition (Figure 30).To confirm the effects of *Lacticaseibacillus paracasei* m.biome *LIVESKIN88* (DSM 33788) on the intracellular levels of ROS, HaCaT keratinocytes treated with probiotic suspension were induced with H₂O₂ (Figure 31). Afterwards, the ROS levels were analyzed with a DCF-DA solution. In the untreated cells, H₂O₂ at a concentration of 1500 µmol/L (Ctrl +) significantly increased the intracellular ROS levels in the HaCaT keratinocytes. Cells treated with *Lacticaseibacillus paracasei* m.biome *LIVESKIN88* (DSM 33788) showed inhibitory effects on H₂O₂-induced ROS. The evidence suggests that *Lacticaseibacillus paracasei* m.biome *LIVESKIN88* (DSM 33788) protects HaCaT keratinocytes by scavenging the ROS produced in response to H₂O₂ exposure.

### Effect of Lacticaseibacillus paracasei m.biome LIVESKIN88 (DSM 33788) against S. aureus infection

Antimicrobial activity against pathogens is another important attribute to be considered in the selection of potential probiotic strains for maintaining a healthy microbial balance in the skin.

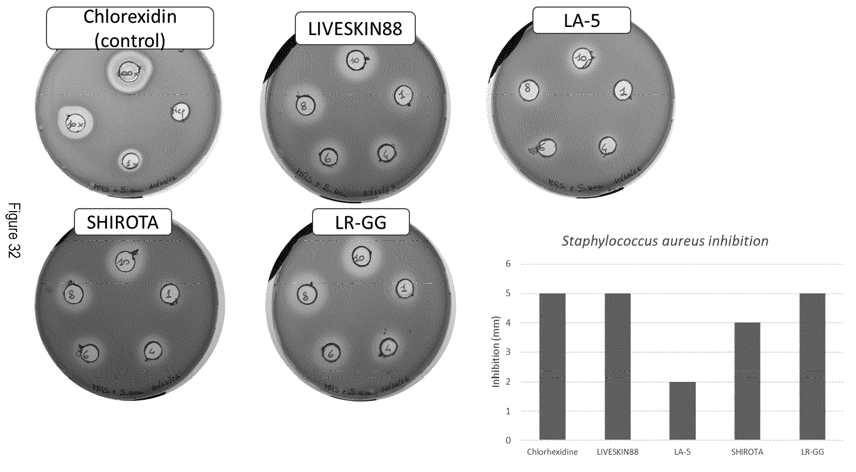

In the present analysis, five *Lactobacillus* strains have been analysed and all showed high antagonistic activity against *S*. *aureus* pathogen strain, which are pathogenic for human skin. *Lacticaseibacillus paracasei* m.biome *LIVESKIN88* (DSM 33788) showed high antagonistic effects against the tested pathogen strain, showing higher growth inhibition ability than the reference strain *L. casei* Shirota (Figure 32).

### CONCLUSION

The data obtained shown the synergistic effect of HA mask and *Lacticaseibacillus paracasei* m.biome *LIVESKIN88* (DSM 33788): mask+probiotic has shown that the probiotic *Lacticaseibacillus paracasei* m.biome *LIVESKIN88* (DSM 33788) has a positive effect on HaCaT epithelization, and, at the same time, the HA has a positive effect on *Lacticaseibacillus paracasei* m.biome *LIVESKIN88* (DSM 33788) adhesive property.

The same synergistic effect was showed in the regulation of NFkB expression, highlighting excellent immunomodulatory activity of the probiotic product. Lacticaseibacillus paracasei m.biome LIVESKIN88 (DSM 33788) showed also antioxidant property through a strong scavenging effect against ROS normally produced by skin aging. Moreover, Lacticaseibacillus paracasei m.biome LIVESKIN88 (DSM 33788) show a strong antimicrobial effect against S. aureus, and is therefore a valuable agent for the treatment and prevention of infections.

## Claims

1. A strain of bacteria for use in the preventive or curative treatment of skin infections and/or skin inflammations caused by *Streptococcus aureus;* wherein said strain is *Lacticaseibacillus paracasei* m.biome LIVESKIN88 deposited with the Deutsche Sammlung von Mikroorganismen und Zellkulturen GmbH (DSMZ) as *Lactobacillus paracasei* under deposit number DSM 33788.

2. The cosmetic use of *Lacticaseibacillus paracasei* m.biome LIVESKIN88 deposited with the Deutsche Sammlung von Mikroorganismen und Zellkulturen GmbH (DSMZ) as *Lactobacillus paracasei* under deposit number DSM 33788 to reduce the skin oxidative stress condition.

3. A composition comprising *Lacticaseibacillus paracasei* m.biome LIVESKIN88 deposited with the Deutsche Sammlung von Mikroorganismen und Zellkulturen GmbH (DSMZ) as *Lactobacillus paracasei* under deposit number DSM 33788 and optionally at least a pharmaceutically acceptable excipient, for use in the preventive or curative treatment of skin infections and/or skin inflammations caused by *Streptococcus aureus.*

4. A composition comprising *Lacticaseibacillus paracasei* m.biome LIVESKIN88 deposited with the Deutsche Sammlung von Mikroorganismen und Zellkulturen GmbH (DSMZ) as Lactobacillus paracasei under deposit number DSM 33788 and hyaluronic acid or salts thereof, and optionally at least a pharmaceutically acceptable excipient, for use in wound healing.

5. The non-therapeutic use of a dermatological or cosmetic composition comprising
- *Lacticaseibacillus paracasei* m.biome LIVESKIN88 deposited with the Deutsche Sammlung von Mikroorganismen und Zellkulturen GmbH (DSMZ) as *Lactobacillus paracasei* under deposit number DSM 33788; and optionally
- hyaluronic acid or salts thereof;
in preventing skin ageing and/or in the regeneration process of human skin and/or to stimulate skin barrier and metabolic functions.

## Patentansprüche

1. Bakterienstamm zur Verwendung bei der präventiven oder kurativen Behandlung von Hautinfektionen und/oder Hautentzündungen, die durch *Streptococcus aureus* verursacht werden; wobei der Stamm *Lacticaseibacillus paracasei* m.biome LIVESKIN88 ist, der bei der Deutschen Sammlung von Mikroorganismen und Zellkulturen GmbH (DSMZ) als *Lactobacillus paracasei* unter der Hinterlegungsnummer DSM 33788 hinterlegt ist.

2. Kosmetische Verwendung von *Lacticaseibacillus paracasei* m.biome LIVESKIN88, hinterlegt bei der Deutschen Sammlung von Mikroorganismen und Zellkulturen GmbH (DSMZ) als *Lactobacillus paracasei* unter der Hinterlegungsnummer DSM 33788, zur Verringerung des oxidativen Stresszustandes der Haut.

3. Zusammensetzung, umfassend *Lacticaseibacillus paracasei* m.biome LIVESKIN88, hinterlegt bei der Deutschen Sammlung von Mikroorganismen und Zellkulturen GmbH (DSMZ) als *Lactobacillus paracasei* unter der Hinterlegungsnummer DSM 33788, und optional mindestens einen pharmazeutisch verträglichen Hilfsstoff zur Verwendung bei der präventiven oder kurativen Behandlung von Hautinfektionen und/oder Hautentzündungen, die durch *Streptococcus aureus* verursacht werden.

4. Zusammensetzung, umfassend *Lacticaseibacillus paracasei* m.biome LIVESKIN88, hinterlegt bei der Deutschen Sammlung von Mikroorganismen und Zellkulturen GmbH (DSMZ) als Lactobacillus paracasei unter der Hinterlegungsnummer DSM 33788, und Hyaluronsäure oder Salze davon, und optional mindestens einen pharmazeutisch verträglichen Hilfsstoff zur Verwendung bei der Wundheilung.

5. Nicht-therapeutische Verwendung einer dermatologischen oder kosmetischen Zusammensetzung, umfassend:
- *Lacticaseibacillus paracasei* m.biome LIVESKIN88, hinterlegt bei der Deutschen Sammlung von Mikroorganismen und Zellkulturen GmbH (DSMZ) als *Lactobacillus paracasei* unter der Hinterlegungsnummer DSM 33788; und optional
- Hyaluronsäure oder Salze davon;
zur Prävention der Hautalterung und/oder im Regenerationsprozess der menschlichen Haut und/oder zur Stimulierung der Hautbarriere und der Stoffwechselfunktionen.

## Revendications

1. Souche bactérienne destinée à être utilisée dans le traitement préventif ou curatif d'infections cutanées et/ou d'inflammations cutanées causées par *Streptococcus aureus* ; dans laquelle ladite souche est *Lacticaseibacillus paracasei* m.biome LIVESKIN88, déposée auprès de la Deutsche Sammlung von Mikroorganismen und Zellkulturen GmbH (DSMZ) identifiée comme *Lactobacillus paracasei* sous le numéro d'accès DSM 33788.

2. Utilisation cosmétique de *Lacticaseibacillus paracasei* m.biome LIVESKIN88, déposée auprès de la Deutsche Sammlung von Mikroorganismen und Zellkulturen GmbH (DSMZ) identifiée comme *Lactobacillus paracasei* sous le numéro d'accès DSM 33788, pour réduire l'état de stress oxydatif cutané.

3. Composition comprenant *Lacticaseibacillus paracasei* m.biome LIVESKIN88, déposée auprès de la Deutsche Sammlung von Mikroorganismen und Zellkulturen GmbH (DSMZ) identifiée comme *Lactobacillus paracasei* sous le numéro d'accès DSM 33788, et éventuellement au moins un excipient pharmaceutiquement acceptable, destinée à être utilisée dans le traitement préventif ou curatif d'infections cutanées et/ou d'inflammations cutanées causées par *Streptococcus aureus.*

4. Composition comprenant *Lacticaseibacillus paracasei* m.biome LIVESKIN88, déposée auprès de la Deutsche Sammlung von Mikroorganismen und Zellkulturen GmbH (DSMZ) identifiée comme *Lactobacillus paracasei* sous le numéro d'accès DSM 33788, et de l'acide hyaluronique ou ses sels, et éventuellement au moins un excipient pharmaceutiquement acceptable, destinée à être utilisée dans la cicatrisation des plaies.

5. Utilisation non thérapeutique d'une composition dermatologique ou cosmétique, comprenant :
- *Lacticaseibacillus paracasei* m.biome LIVESKIN88, déposée auprès de la Deutsche Sammlung von Mikroorganismen und Zellkulturen GmbH (DSMZ) identifiée comme *Lactobacillus paracasei* sous le numéro d'accès DSM 33788 ; et éventuellement
- de l'acide hyaluronique ou ses sels ;
dans la prévention du vieillissement cutané et/ou dans le processus de régénération de la peau humaine et/ou pour stimuler la barrière cutanée et des fonctions métaboliques.
